# EUROPÄISCHE PATENTSCHRIFT

(11) **EP 2 697 729 B1**
(45) Veröffentlichungstag und Bekanntmachung des Hinweises auf die Patenterteilung: **04.05.2022**
(21) Anmeldenummer: 12719256.5
(22) Anmeldetag: 10.04.2012
(51) Int. Cl.: G16H 50/20, G16H 50/70

(54) **VERFAHREN UND SYSTEM ZUR UNTERSTÜTZUNG DER DIAGNOSE EINES WENIGSTENS EINE STÖRUNG AUFWEISENDEN OBJEKTES**
METHOD AND SYSTEM FOR SUPPORTING THE DIAGNOSIS OF AN OBJECT HAVING AT LEAST ONE DISTURBANCE
PROCÉDÉ ET SYSTÈME D'ASSISTANCE AU DIAGNOSTIC D'UN OBJET PRÉSENTANT AU MOINS UNE PERTURBATION

(30) Priorität: 11.04.2011 DE 102011016691
(43) Veröffentlichungstag der Anmeldung: 19.02.2014
(73) Patentinhaber: Ada Health GmbH, 10997 Berlin (DE)
(72) Erfinder: MÜLLER, Albrecht, 82057 Icking (DE); HIRSCH, Martin, 35037Marburg (DE)
(74) Vertreter: BSB Patentanwälte Schütte & Engelen Partnerschaft mbB
(86) Internationale Anmeldenummer: PCT/EP2012/001553
(87) Internationale Veröffentlichungsnummer: WO 2012/139749

(56) Entgegenhaltungen:
- WO-A2-2008/106665
- US-A1- 2006 061 589
- US-A1- 2009 094 053

## Beschreibung

Die vorliegende Erfindung betrifft ein Computer-implementiertes Verfahren und ein System zur Unterstützung der Diagnose einer Krankheit und zur Auswahl eines die Krankheit wenigstens teilweise charakterisierenden Befundes, wobei auf eine Anzahl von in wenigstens einer Datenbank gespeicherten Befunden zurückgegriffen wird.

Somit betrifft die Erfindung z. B. ein Verfahren zur Unterstützung von Ärzten bei der Diagnose von Krankheiten. Dabei wird dem Arzt nicht die Entscheidung der Diagnose abgenommen, sondern es wird nach der Eingabe von Symptomen eine Auswahl von Diagnosen angezeigt, die mit den Symptomen des Patienten und deren Bewertung durch den behandelnden Arzt am vergleichbarsten sind.

Ebenso werden nachfolgend zur besseren Veranschaulichung der Funktionsweise der Erfindung rein illustrativ jedoch nicht gemäß der vorliegenden Erfindung ein Verfahren und ein System beschrieben, bei dem ein Experte analog zu dem erfindungsgemäßen Verfahren und dem erfindungsgemäßen System bei der Analyse eines wenigstens eine Störung aufweisenden komplexen Objektes unterstützt wird.

Im Stand der Technik sind verschiedene Verfahren und Systeme zur Unterstützung bei der Auswahl von Befunden bekannt geworden. Beispielsweise kann ein Arzt in einer Datenbank alle Symptome eines Patienten eingeben, wobei er eine Liste der Krankheiten mit genau diesen Symptome erhält.

Aus der US 2009/0094053 A1 ist ein Verfahren bekannt geworden, bei dem mehrere Symptome eines Patienten in ein System eingegeben werden und bei dem die Symptome grafisch in einem Netzdiagramm dargestellt werden. Das System führt eine Diagnose durch und zeigt die wahrscheinlichsten Krankheiten mit der höchsten Übereinstimmung ebenfalls in Netzdiagrammen an.

Ebenso ist es beispielsweise bei der Diagnose von Störungen an komplexen technischen Anlagen möglich, alle unregelmäßigen Messwerte und Betriebsbedingungen einzugeben, woraufhin eine Auswahl der möglichen Ursachen geliefert wird.

All diese Verfahren und Systeme funktionieren an sich zuverlässig. Nachteilig daran ist aber, dass es beispielsweise bei der Diagnose von Krankheiten möglich ist, dass ein Patient an einer atypischen Form einer Krankheit leidet, die ein an sich typisches Merkmal (Symptom) nicht aufweist, und die noch nicht beschrieben wurde. Dadurch kann die Diagnose irregeführt werden, denn der Arzt wird gegebenenfalls diese Krankheit nicht mehr in Betracht ziehen. Bei den bekannten Verfahren wird diese Krankheit aufgrund des Fehlens eines an sich typischen Merkmals nicht mehr angezeigt. Ein weiteres Problem bei der Diagnose kann sich dann ergeben, wenn der Patient an zwei Krankheiten leidet, deren Symptome sich überschneiden. Eine Datenbankabfrage mit allen Symptomen bringt dann kein Ergebnis. Ein weitere Problem bestehender Verfahren besteht darin, dass das "Bauchgefühl" eines Arztes für die Wichtigkeit eines Symptoms in Hinblick auf das Krankheitsgeschehen nicht berücksichtigt wird. So ist z. B. Erbrechen im Kontext einer Schwindelerkrankung weniger wichtig (da meist eine Begleiterscheinung) als im Kontext eines blutigen Stuhlgangs.

Auch bei der Diagnose beispielsweise eines defekten Kraftfahrzeugs sind die Merkmale des aktuellen Störungszustandes nicht alle unbedingt wesentlich. Der mit der Reparatur betraute Experte wird aufgrund seiner Erfahrung wissen, dass eine Beule am Kotflügel keine Bedeutung für einen zerschlissenen Sitzbezug hat. Ein Klopfen des laufenden Motors könnte aber eine fehlerhafte Motorsteuerung zur Ursache haben. Die Ursache könnte aber auch ein versehentlich fehlerhaft getankter Treibstoff sein.

Bei komplexen technischen Produkten können deshalb auch mehrere Fehler vorliegen, die nichts miteinander zu tun haben, was die Diagnose erschwert.

Es ist deshalb die Aufgabe der Erfindung, ein Verfahren und ein System zur Unterstützung bei Diagnose einer Krankheit eines Patienten zur Verfügung zu stellen, welches dem Benutzer eine übersichtliche und zuverlässige Auswahlfunktion ermöglicht.

Diese Aufgabe wird gelöst durch ein Verfahren mit den Merkmalen des Anspruchs 1 und durch ein System mit den Merkmalen des Anspruchs 15. Bevorzugte Weiterbildungen der Erfindung sind Gegenstand der Unteransprüche. Weitere Vorteile und Merkmale der vorliegenden Erfindung ergeben sich aus der allgemeinen Beschreibung und der Beschreibung eines Ausführungsbeispiels.

Die nachfolgenden Ausführungen beschreiben die Erfindung teilweise auch anhand der Diagnose eines wenigstens eine Störung aufweisenden Objektes und der Auswahl eines den Objektzustand wenigstens teilweise charakterisierenden Befundes. Dabei werden folgende Begriffe geleichbedeutend mit Bezug auf die Diagnose von Krankheiten verwendet: Objekt = Patient, Objektzustand = Krankheit, Merkmal = Symptom, Merkmalskonstellation = Symptomkonstellation, aktuelle Merkmalskonstellation = Krankheitsbild. Alle offenabarten Merkmale gelten gleichbedeutend auch für das erfindungsgemäße Verfahren und das erfindungsgemäße System.

Das erfindungsgemäße Computer-implementierte Verfahren dient zur Unterstützung der Diagnose eines wenigstens eine Störung aufweisenden Objektes und zur Auswahl eines den Objektzustand wenigstens teilweise charakterisierenden Befundes, wobei in wenigstens einer Datenbank eine Anzahl von Befunden gespeichert ist, auf welche unterstützend zurückgegriffen werden kann. Jeder gespeicherte Befund wird durch eine Schwere oder Gefährlichkeit des Befundes und durch eine Merkmalskonstellation mehrerer Merkmale beschrieben. Die Schwere eines Befundes ist insofern kein Merkmal oder Symptom, sondern gibt z. B. die Gefährlichkeit der Erkrankung oder der Störung an. Eine graphische Wiedergabe der Merkmalskonstellation definiert ein visuelles Störungsbild des Befundes. Jedes Merkmal weist wenigstens einen Merkmalstyp und wenigstens eine Ausprägung auf. In jedem Störungsbild wird jedes Merkmal durch ein Symbol repräsentiert, dessen Koordinaten in einer ersten Dimension durch den Merkmalstyp des Merkmals und in einer zweiten Dimension durch die Ausprägung des Merkmals bestimmt werden.

In dem Verfahren erfolgt eine dialogbasierte oder dialogunterstützte visuelle Modellierung des Objektzustandes. Der jeweils aktuelle Objektzustand wird durch eine aktuelle Merkmalskonstellation beschrieben, die ein Merkmal oder insbesondere mehrere Merkmale umfassen kann. Jedes Merkmal weist wenigstens einen Merkmalstyp und wenigstens eine Ausprägung auf. Die aktuelle Merkmalskonstellation definiert ein Objektbild, wobei in dem Objektbild jedes Merkmal durch ein Symbol repräsentiert wird, dessen Koordinaten in einer ersten Dimension durch den Merkmalstyp des Merkmals und in einer zweiten Dimension durch die Ausprägung des Merkmals bestimmt werden. Das bedeutet, dass das Objektbild und das Störungsbild grundsätzlich nach gleichen Vorschriften aufgebaut werden, sodass sich visuell vergleichbare Bilder ergeben.

Zur Unterstützung der Diagnose sind wenigstens eine insbesondere mobile Recheneinrichtung, wenigstens eine Vergleichseinrichtung, wenigstens eine Eingabeeinrichtung und wenigstens eine Anzeigeeinrichtung vorgesehen. Die Vergleichseinrichtung kann Teil der Recheneinrichtung sein oder aber separat ausgebildet sein.

Es werden insbesondere zum Start die folgenden Verfahrensschritte durchgeführt:
- Aus einer vordefinierten Merkmalskonstellation wird eine aktuelle Merkmalskonstellation abgeleitet,
- aus einer vordefinierten Befundliste mit gespeicherten Befunden wird eine aktuelle Befundliste abgeleitet, und
- aus einer vordefinierten Vorschlagsliste für Merkmale wird eine aktuelle Vorschlagsliste für Merkmale abgeleitet,

Die folgenden Verfahrensschritte werden in dieser oder in einer anderen geeigneten Reihenfolge vorzugsweise in einer Schleife automatisch wiederholt, bis ein Befund manuell festgelegt oder eingegeben wird oder bis ein Störungsbild und damit der zugehörige Befund ausgewählt wird oder bis das Verfahren abgebrochen wird:
a) Mit der insbesondere interaktiven, mobilen und vorzugsweise gestengesteuerten Eingabeeinrichtung wird wenigstens ein Merkmal der aktuellen Merkmalskonstellation vorzugsweise gestengesteuert modifiziert, um den Objektzustand zu modellieren. Die derart modifizierte Merkmalskonstellation wird als aktuelle Merkmalskonstellation gespeichert. Die aktuelle Merkmalskonstellation wird als aktuelles Objektbild automatisch auf der Anzeigeeinrichtung graphisch ausgegeben. Praktisch bedeutet das, dass die aktuelle Merkmalskonstellation als aktuelles Objektbild interaktiv visuell vom Anwender erzeugt wird.
b) Die Vergleichseinrichtung vergleicht nach jeder Modifizierung eines Merkmals unmittelbar und automatisch die aktuelle Merkmalskonstellation mit den gespeicherten Merkmalskonstellationen der Befunde der aktuellen Befundliste und präsentiert die Ergebnisse des Vergleichs unmittelbar auf der Anzeigeeinrichtung und somit im Blickfeld des Anwenders. Diese Unmittelbarkeit der Präsentation der Ergebnisse des Vergleichs ist ein wesentlicher Bestandteil des Systems, da nur diese Unmittelbarkeit eine dialogische Beziehung zwischen Anwender und System herstellt, die notwendig ist, damit das System Anregungen ausgibt und z.B. in den aktuellen diagnostischen Gedanken des Anwenders hinein neue Informationen situationsbedingt einspielen kann. Die Unmittelbarkeit ist auch deshalb von wesentlicher Bedeutung, da nur durch sie eine Art von Wenn-dann Simulationen spielerisch möglich wird. Der Experte kann testhalber ein Merkmal "mal eben" mit einer Geste modifizieren, um zu schauen, welche Referenz-Objekte dann angezeigt werden. Das ist ein erheblicher Vorteil des erfinderischen Verfahrens, da es ein solch spielerisch experimentelles, intuitives Vorgehen ermöglicht und fördert.
c) Die Vergleichseinrichtung vergleicht nach jeder Modifizierung eines Merkmals unmittelbar und automatisch die aktuelle Merkmalskonstellation mit den gespeicherten Merkmalskonstellationen der Befunde der aktuellen Befundliste und entfernt aus der aktuellen Befundliste unmittelbar jene Befunde, deren Übereinstimmung mit der aktuellen Merkmalskonstellation ein vorbestimmtes erstes Maß unterschreiten. Der Vergleich kann z. B. als graphischer Vergleich oder numerisch erfolgen.
d) Die Vergleichseinrichtung vergleicht nach jeder Modifizierung eines Merkmals automatisch die aktuelle Merkmalskonstellation mit den Merkmalskonstellationen von den in der Datenbank gespeicherten Befunden und fügt der aktuellen Befundliste unmittelbar gespeicherte Befunde hinzu, bei denen die Übereinstimmung der jeweiligen Merkmalskonstellation mit der aktuellen Merkmalskonstellation ein vorbestimmtes zweites Maß überschreitet. Der Vergleich kann z. B. auch als graphischer Vergleich oder numerisch erfolgen.
e) Die aktuelle Befundliste wird nach jeder Modifizierung eines Merkmals mit der Recheneinrichtung unmittelbar und automatisch sortiert, wobei die Ähnlichkeit der aktuellen Merkmalskonstellation mit der jeweiligen Merkmalskonstellation der gespeicherten Befunde und die Schwere des gespeicherten Befundes bei der Sortierung berücksichtigt werden.
f) Von wenigstens einem Befund der aktuellen Befundliste wird die zugehörige Merkmalskonstellation als Störungsbild auf der Anzeigeeinrichtung aufgetragen, sofern die aktuelle Befundliste überhaupt einen relevanten Befund enthält. Insbesondere werden von zwei, drei oder mehr der relevantesten oder ähnlichsten Befunde der aktuellen Befundliste die jeweiligen Merkmalskonstellationen als Störungsbild aufgetragen.
g) Die Vergleichseinrichtung vergleicht nach jeder Modifizierung eines Merkmals automatisch die aktuelle Vorschlagsliste für Merkmale mit den Merkmalen der Befunde der aktuellen Befundliste und sortiert die aktuelle Vorschlagsliste für Merkmale neu. Die aktuelle Vorschlagsliste wird gespeichert und unmittelbar ausgegeben.

Das erfindungsgemäße Verfahren hat viele Vorteile. Mit Hilfe der visuell interaktiven und insbesondere mobilen Eingabe von den Objektzustand charakterisierenden Merkmalen wird der Experte beim Finden eines Befundes vor Ort unterstützt. Dabei geht es hier darum, dem jeweiligen Experten Anregungen und Hilfestellungen zur Diagnose unmittelbar am Ort des Geschehens, also z. B. am Krankenbett, zu geben. Die Diagnose selbst bleibt dabei stets dem Experten selbst vorbehalten.

Bei bestimmten Störungen von beispielsweise komplexen technischen Anlagen weiß der Experte, wenn er das Geräusch z. B. eines Motors hört, dass etwas nicht in Ordnung ist, ohne aber direkt die Ursache des Problems zu kennen. Die Erfindung beschäftigt sich nun nicht primär mit der Suche nach Befunden mit ähnlichen Symptomen, sondern damit, dem Experten aufgrund eines systematischen visuell unterstützten Vergleichs unmittelbar und vor Ort Hinweise auf weitere Merkmale und gegebenenfalls Untersuchungen zu geben, die schließlich zur Bestimmung eines Befundes und somit zur Kenntnis des zugrundeliegenden Problems und möglicher Aktionen zur Behebung der Störung führen. Es geht also insbesondere darum, beim Experten den Einfall zu locken, nicht aber, ihm die Diagnose abzunehmen. Das erfindungsgemäße Verfahren nutzt die Kreativität des Experten und dient als Werkzeug zur Unterstützung der Diagnose, diagnostiziert aber nicht selbst.

Die Erfindung erlaubt eine einfache Visualisierung verschiedener gespeicherter Befunde und Gegenüberstellung mit dem Objektzustand, so wie er bislang eingegeben wurde. Durch visuellen Vergleich erhält der Experte Ideen und Anregungen, welche Merkmale eine hohe Differenzierung erlauben, sodass eine effektive Analyse erfolgen kann.

Bei scharf definierten Auswahlprozessen wie im Stand der Technik ist es oftmals mit einer boolschen Verschaltung der Symptome oder durch Traversieren eines Entscheidungsbaumes schwierig, das korrekte Ergebnis zu erhalten. Die vorliegende Erfindung gibt keinen Entscheidungsbaum und/oder einen festen Ablauf vor, sondern gibt aufgrund eines im Hintergrund ablaufenden Rechenprozesses dem das System benutzenden Experten ähnliche Befunde vor und zeigt relevante Merkmale an, die Ideen und Anregungen zur Findung der Lösung geben.

Ein erheblicher Vorteil des erfindungsgemäßen Verfahrens besteht darin, dass Merkmalskonstellationen graphisch interaktiv verarbeitet werden, so dass sie visuell einfach und schnell miteinander verglichen und vorzugsweise mit wenigen Gesten modifiziert werden können.

Es ist möglich, dass die vordefinierte Merkmalskonstellation eine "leere" Merkmalskonstellation ist, der also zunächst ein leeres Objektbild zugeordnet ist. Wenn eine solche vordefinierte Merkmalskonstellation zu Beginn des Verfahrens als aktuelle Merkmalskonstellation vorgegeben wird, wird bei der Diagnose bei "Null" gestartet. Es kann aber auch sein, dass als vordefinierte Merkmalskonstellation eine schon zuvor abgespeicherte Merkmalskonstellation dient, die beispielsweise zuvor gespeichert wurde.

Das Verfahren sieht insbesondere auch eine Speichermöglichkeit vor, sodass das Verfahren jederzeit wieder aufgenommen und fortgesetzt werden kann.

Auch die vordefinierte Befundliste kann eine "leere" oder zuvor gefüllte Befundliste sein, die bei Start des Verfahrens zugrunde gelegt wird.

Die vordefinierte Vorschlagsliste für Merkmale kann alle Merkmale umfassen und kann zunächst irgendwie sortiert sein.

Jede Merkmalskonstellation wird durch eine Reihe von Merkmalen definiert, die jeweils einen Merkmalstyp und eine entsprechende Ausprägung ausweisen. Dabei ist es ein wichtiger Punkt der Erfindung, dass jedes Merkmal Informationen über seine Wichtigkeit in Hinblick auf die Störungsursache trägt und anzeigt. Beispielsweise tritt bei einem bestimmten Krankheitsbild ein Symptom auf, das von erfahrenen Ärzten als nicht besonders relevant oder aber grade als besonders charakteristisch angesehen wird (sogenannte Leitsymptome). Demzufolge kann die Wichtigkeit eines Merkmals weniger stark oder stärker ausgebildet sein bzw. bewertet werden. Aufgrund dieses Umstandes können bei dem erfindungsgemäßen Verfahren zum Beispiel relevante Merkmale in einem Kreis zentrumsnah, weniger relevante Merkmale zentrumsfern angeordnet werden, was der intuitiven Interpretation durch den Experten zuträglich ist.

Ein erheblicher Vorteil des erfindungsgemäßen Verfahrens besteht auch darin, dass es kognitiv gegen Merkmalsschwankungen robust ist. Durch die graphische Darstellung der Merkmalskonstellationen und durch den Vergleich der Merkmalskonstellation mit den gespeicherten Merkmalskonstellationen kann der Benutzer einfach und schnell graphisch visuell überblicken, welche der gespeicherten Merkmalskonstellationen im vorliegenden Fall zur Auswahl geeignet sind, und wie sie sich unterscheiden. Dadurch kann die Auswahl möglicher Befunde sehr schnell sehr stark eingeschränkt werden, sodass nur relevante auszuwählende Objekte übrig bleiben, mit denen sich der Benutzer intensiver auseinandersetzen kann.

Es sind wenigstens einen Darstellungsbereich und wenigstens einen Auswahlbereich vorgesehen. Der Darstellungsbereich und der Auswahlbereich können auf einer gemeinsamen Anzeigeeinrichtung oder auf voneinander getrennten Anzeigeeinrichtungen angeordnet werden. Die Ausgabe des Objektbildes erfolgt in dem Darstellungsbereich und die Ausgabe des wenigstens einen Störungsbildes erfolgt in dem Auswahlbereich.

Mit der Eingabeeinrichtung kann bei jedem Verfahrensschritt wenigstens ein Merkmal des Objektzustandes modifiziert, d. h. insbesondere eingegeben, entfernt oder verändert werden. Nach der Modifizierung wird die aktuelle Merkmalskonstellation gespeichert.

Vorzugsweise erfolgt eine Modifizierung oder Eingabe wenigstens eines Merkmales der aktuellen Merkmalskonstellation durch eine graphisch interaktive Eingabe. Beispielsweise kann die Eingabe über Wischgesten erfolgen.

Dadurch, dass in dem Auswahlbereich wenigstens ein gespeichertes Befund bzw. dessen Merkmalskonstellation graphisch dargestellt wird, kann durch einfachen Vergleich dieses graphischen Musters mit dem graphischen Muster im Darstellungsbereich ein für das menschliche Gehirn sehr effizienter visueller Vergleich erfolgen, der mit hoher Zuverlässigkeit die Auswahl eines oder mehrerer gespeicherter Befunde ermöglicht. Dabei muss insbesondere nicht jedes einzelne Merkmal miteinander verglichen werden, sondern es kann einfach über einen graphischen Mustervergleich eine recht zuverlässige Abschätzung erfolgen, welches gespeicherte Muster mit der aktuell zu bestimmenden Merkmalskonstellation am besten übereinstimmt. Dazu werden die gespeicherten Befunde im Auswahlbereich genauso graphisch aufbereitet, wie die Merkmale des zu bestimmenden Objektzustandes im Darstellungsbereich.

Insbesondere muss dabei nicht in einer Tabellenform oder dergleichen eine textuelle Beschreibung jedes einzelnen Merkmals mit den beschreibenden Texten der gespeicherten Objekte verglichen werden.

Wird bei der Diagnose einer Krankheit jedes Symptom des Patienten durch ein Symbol in bzw. auf dem Darstellungsbereich repräsentiert, so ergibt die Gesamtzahl der Symbole des Patienten insgesamt ein für den Zustand des Patienten charakteristisches graphisches Muster bzw. eine Merkmalskonstellation. Dabei wird jedes Symbol insbesondere bezüglich einer ersten Koordinate durch den Typ bzw. durch die Art des Symptoms gekennzeichnet, während die zweite Dimension bzw. die zweite Koordinate zur Anordnung des zugehörigen Symbols definiert die Wichtigkeit des entsprechenden Symptoms . Es ist ein wesentlicher Bestandteil des erfindungsgemäßen Verfahrens, das hier nicht ein objektiv metrischer Aspekt (wie z. B. der Temperaturwert einer Fiebermessung) positionsbestimmend ist, sondern ein subjektiv einschätzender Aspekt. Dadurch inkludiert das Verfahren "das Bauchgefühl" des Experten in den Vergleichsprozess und eben darum geht es dem erfindungsgemäßen Verfahren auch.

Hat der Patient beispielsweise Kopfschmerzen, so kann als Merkmalstyp der Typus "Kopfschmerzen" gewählt werden. Unter dem Begriff "Wichtigkeit bzw. "Relevanz" wird im Sinne dieser Anmeldung verstanden, ob das Merkmal für das Krankheitsgeschehen von zentraler Bedeutung oder eher eine Begleiterscheinung ist. Unter "Intensität" oder "Ausprägung" wird vorzugsweise der Ausprägungsgrad des entsprechenden Merkmals angesehen (Wie stark sind die Kopfschmerzen auf einer Schmerzskala?).

Bei anderen Objekten, wie beispielsweise Gegenständen wie Kraftfahrzeugen und dergleichen, kann der Merkmalstyp beispielsweise ein Klopfen des Motors bezeichnen, während die Ausprägung je nach sonstiger Konstellation wichtig (= z. B. groß) oder unwichtig sein kann. Bei der Beurteilung von verschissenen Sitzbezügen ist das Klopfen des Motors von untergeordneter Wichtigkeit, während bei der Beurteilung von Motorproblemen ein solches Merkmal eine deutlich höhere Ausprägung haben wird.

In allen Ausgestaltungen ist es besonders bevorzugt, dass ein Koordinatensystem gewählt wird, bei dem der Merkmalstyp über den Winkel aufgetragen wird, während die Wichtigkeit (Relevanz) eine Funktion des Radius bzw. des Abstandes von einem zentralen Punkt oder einer Achse ist, und die Intensität bzw. der Ausprägungsgrad durch die Größe des Symbols zum Ausdruck gebracht wird. Möglich ist es dabei, dass mit zunehmender Wichtigkeit der Radius, also die entsprechende zweite Koordinate, geringer wird, während mit abnehmender Bedeutung und somit abnehmender Wichtigkeit der Radius größer wird. Beispielsweise kann der Darstellungsbereich und/oder der Auswahlbereich durch eine Fläche definiert werden, wobei unterschiedliche Symptome, also unterschiedliche Merkmalstypen, über den Winkel aufgetragen werden und eine besonders starke Wichtigkeit zentrumsnah abgebildet wird. Durch die Zuordnung des Merkmalstyps zum Winkel wird gewährleistet, dass beispielsweise Kopfschmerzen, Gliederschmerzen oder Bauchschmerzen jeweils an einer unterschiedlichen Winkelposition angeordnet werden, während die Winkelposition eines bestimmten Symptoms jedes Mal die gleiche ist. Wenn es sich um die Diagnose einer Krankheit handelt, werden Kopfschmerzen also regelmäßig an der gleichen Winkelposition eingezeichnet was die Wiedererkennbarkeit wesentlich verbessert. Die Wichtigkeit des entsprechenden Merkmals im pathologischen Geschehen wird durch seine Zentrumsnähe beschrieben, sodass zentrumsnah Symbole angeordnet werden, die beispielsweise nach Auffassung und Beurteilung des behandelnden Arztes eine hohe Relevanz des entsprechenden Merkmals für die aktuelle Diagnose haben. Die Intensität bzw. der Ausprägungsgrad des Merkmals wird durch die Größe des Symbols definiert bzw. codiert.

Möglich ist es aber auch, dass ein X-Y-Diagramm erstellt wird, bei dem die X-Koordinate den Merkmalstyp wiedergibt und bei dem auf der Y-Koordinate die Relevanz wiedergegeben wird. Dabei kann eine besonders starke Relevanz einem großen Y-Wert oder einem kleinen Y-Wert entsprechen. Auch bei einer solchen Darstellung ist ein graphisches Muster mehrerer Merkmale charakteristisch für einen bestimmten Befund. Dadurch kann über einen einfachen Mustervergleich eine Konzentration auf einige wenige gespeicherte Befunde erfolgen.

Das grundlegende Ziel des erfindungsgemäßen Verfahrens bei dem Einsatz bei der Unterstützung von medizinischen Diagnosen ist es nicht, den Arzt zu ersetzen und ein einfaches Diagnosesystem zu erhalten, sondern den erfahrenen Arzt bei der Diagnose zu unterstützen, dahin gehend, dass man ihm Krankheitsbilder und Symptome in Erinnerung ruft, die zur aktuellen Merkmalskonstellation passen, in der Praxis aber relativ selten vorkommen, und so vom Arzt schnell vergessen oder übersehen werden. So kann z. B. das Symptom "Schwindel" für eine Vielzahl unterschiedlicher Krankheiten und somit Diagnosen stehen. Genauso verhält es sich auch bei dem Symptom "Bauchschmerzen" bei z. B. Kindern. Selbst für einen erfahrenen Facharzt kann es sehr schwierig sein, bei solchen Symptomen eine geeignete Diagnose zu finden, da die Krankheitsbilder unterschiedlich viele Symptome in unterschiedlicher Ausprägung ausweisen. Insbesondere, da solche Symptome auch bei sogenannten Seltenen Krankheiten auftauchen können, die ein Arzt - selbst als Facharzt - nur selten oder nie in seiner Berufspraxis erleben wird, ist es verständlicherweise schwer, eine zutreffende Diagnose zu treffen.

Die Erfindung ermöglicht hier über einen Mustervergleich den Vergleich unterschiedlicher Merkmalskonstellationen, sodass im Falle der Diagnose von Krankheiten der Arzt auch Merkmalskonstellationen angezeigt bekommt, die selten sind und dennoch eine hohe Übereinstimmung mit der aktuellen Merkmalskonstellation haben können.

Dabei ist es auch in diesem Beispiel wichtig, dass das erfindungsgemäße Verfahren die subjektiv empfundene Wichtigkeit eines Merkmals durch den behandelnden Arzt erfasst und abbildet.

Das gleiche gilt für komplexe technische Anlagen, bei denen einzelne Störungen unterschiedlichste Ursachen haben können.

Dabei kann der Befund schwerwiegend sein oder nur eine untergeordnete Bedeutung aufweisen. Unübliche Geräusche an einer Turbine in einem Großkraftwerk können z. B. auf defekte Lager hinweisen. Welcher Befund aus Sicht des Experten wie bedeutsam für das störungsverursachende Geschehen ist, soll auch hier erfasst werden. Über ein erfindungsgemäßes Verfahren kann bei solchen Anlagen schnell herausgefunden werden, ob es tatsächlich ein schwerwiegendes Problem gibt oder aber nur einen einfach zu behebenden Schaden mit nur geringem Schadenspotenzial.

In einer bevorzugten Weiterbildung der Erfindung werden in dem Auswahlbereich wenigstens zwei unterschiedliche gespeicherte Merkmalskonstellationen angezeigt. Dabei werden die beiden unterschiedlichen Merkmalskonstellationen jeweils in separaten Fenstern oder Anzeigefeldern oder Bereichen aufgetragen, sodass jede Merkmalskonstellation für sich eigenständig aufgetragen ist. Insbesondere werden drei oder mehr unterschiedliche gespeicherte Merkmalskonstellationen zur Auswahl angezeigt, sodass der Benutzer anhand der Muter der einzelnen Merkmalskonstellationen eine Auswahl treffen kann.

Im Sinne dieser Erfindung kann ein Objektzustand einer Krankheit bzw. einer Diagnose entsprechen, die eine Funktion verschiedener Symptome und somit verschiedener Merkmale ist.

Unmittelbar bei oder aber zumindest direkt nach der Eingabe oder Modifizierung jedes Merkmals wird die angezeigte Auswahl gespeicherter Objekte im Auswahlbereich verändert. Beispielsweise ist es möglich, dass einzelne gespeicherte Merkmalskonstellationen wahrscheinlicher werden, wenn eine größere Anzahl von Merkmalen miteinander übereinstimmt. Dann werden die entsprechenden zugehörigen Befunde bzw. Merkmalskonstellationen bevorzugt angezeigt.

In allen Ausgestaltungen ist es bevorzugt, dass wenigstens ein Merkmal als weitere Eigenschaft eine Intensität aufweist. Wird die Erfindung beispielsweise bei der Diagnose von Krankheiten eingesetzt, kann das Symptom "Kopfschmerzen" nach Auffassung des behandelnden Arztes eine hohe Wichtigkeit für die vorliegende Krankheit aufweisen. Dabei ist es aber möglich, dass die Intensität der "Kopfschmerzen" stark oder auch schwach ist. Folglich bietet sich die Intensität eines Merkmals als weiterer Parameter an. Schwache Kopfschmerzen können in Verbindung mit weiteren Merkmalen sehr prägnant für bestimmte Krankheiten sein.

Vorzugsweise wird die Befundliste also die Reihenfolge der angezeigten Befunde im Auswahlbereich bzw. der Anzeige der gespeicherten Befunde durch das Maß der Unterscheidung eines gespeicherten Befundes zu der eingegebenen Merkmalkonstellation beeinflusst. Das bedeutet, dass eine gespeicherte Merkmalkonstellation die sich stärker von einer angegebenen Merkmalkonstellation unterscheidet als eine andere insbesondere an weniger prominenter Stelle oder später angezeigt wird als eine Merkmalkonstellation, die eine bessere Übereinstimmung aufweist.

Bevorzugt ist beispielsweise die Berechnung der Reihenfolge über die Ermittlung der Abstandsquadrate der einzelnen Symbole zueinander. Insbesondere ist es dabei möglich, dass unterschiedliche Merkmale eine unterschiedliche Gewichtung aufweisen können. Besonders bevorzugt ist es weiterhin, dass die Relevanz und/oder die Ausprägung eines Merkmals in die Gewichtung eingeht. Eine besonders starke Relevanz und/oder Ausprägung erhöht dann die Gewichtung, während eine geringe Relevanz und/oder Ausprägung die Gewichtung verringert.

Besonders stark wird vorzugsweise die Wichtigkeit oder Schwere des Befundes gewichtet. Gefährliche Krankheiten oder Befunde mit hohem Gefährdungspotenzial bekommen eine höhere Priorität als harmlose Krankheiten oder geringfügige technische Probleme.

Vorzugsweise vergleicht die Vergleichseinrichtung nach jeder Modifizierung der aktuellen Merkmalskonstellation die aktuelle Merkmalskonstellation mit den Merkmalskonstellationen von den in der Datenbank gespeicherten Befunden und fügt der aktuellen Befundliste gespeicherte Befunde hinzu, auch wenn die Übereinstimmung der jeweiligen Merkmalskonstellation mit der aktuellen Merkmalskonstellation das vorbestimmte erste Maß unterschreitet, aber ein vorbestimmtes drittes Maß überschreitet, wenn nämlich das Störpotential des gespeicherten Befundes eine vorgegebene Schwelle überschreitet. Das vorbestimmte dritte Maß ist dabei vorzugsweise kleiner als vorbestimmte erste Maß und kann von der Schwere oder dem Störpotential des Befundes abhängen.

Die Befundliste nimmt insbesondere nur einige der möglichen und insbesondere die relevantesten Befunde auf. In einfachen Ausgestaltungen kann die Befundliste jeweils nur die Befunde enthalten, die auf der Anzeigeeinrichtung dargestellt werden. In anderen Ausgestaltungen kann die Befundliste eine Teilmenge der gesamten in der Datenbank enthaltenen Befunde umfassen, von der wiederum nur ein Teil abgebildet wird.

Möglich und bevorzugt ist es auch, dass eine Gewichtung der gespeicherten Objekte vorgenommen wird, die vorgegeben oder dynamisch beeinflussbar ist. Beispielsweise kann bei der Diagnose von Krankheiten eine generelle Gewichtung bestehen, wonach gefährlichere Krankheiten eine höhere Gewichtung aufweisen als weniger gefährliche Krankheiten. Dadurch kann dem behandelnden Arzt ein deutlicher Hinweis auf gefährliche Erkrankungen gegeben werden, die der Arzt durch entsprechende Untersuchung sicher ausschließen kann, um nicht versehentlich eine wesentliche Diagnosemöglichkeit unberücksichtigt zu lassen. Gefährliche Krankheiten werden bevorzugt weiter oben in der Liste angeordnet.

Auf der Anzeigeeinrichtung ist zusätzlich eine Merkmalanzeige vorgesehen, auf der die Vorschlagsliste mit einer Mehrzahl von Merkmalen wiedergegeben wird. Diese Merkmalsanzeige ist insbesondere als Vorschlagsliste ausgebildet und unterstützt den Benutzer bei der Eingabe von Merkmalen. Die einzelnen Merkmale auf der Vorschlagsliste können nach dem Merkmalstyp sortiert vorliegen. Besonders bevorzugt ist es aber, dass die aktuelle Vorschlagsliste dynamisch generiert wird, wobei an vorderen Stellen Merkmale aufgelistet werden, die eine besonders hohe Unterscheidungsmöglichkeit, also einen hohen Informationsgewinn liefern. Gibt es bei dem Einsatz im medizinischen Bereich beispielsweise an Hand der bislang eingegebenen Merkmalkonstellation ein weiteres Merkmal, welches eine hohe Differenzierung ermöglichen würde, so kann es im ergonomischen Sinne sinnvoll sein, zunächst dieses Merkmal als nächstes Merkmal zu überprüfen. So kann dann beispielsweise nach Überprüfung des Vorhandenseins dieses einzelnen Symptoms die Liste der möglichen Erkrankungen erheblich reduziert werden, während andere Symptome nur eine weniger große Differenzierung ermöglichen würden. Deshalb bietet es sich an, die Reihenfolge der einzugebenden Merkmale so vorzugeben, dass eine hohe Relevanz jeder Zeit erzielbar ist. Auf diese Weise wird die Effizienz des Auswahlvorgangs erheblich gesteigert.

Es ist bevorzugt, dass die Vergleichseinrichtung die Merkmale der aktuellen Vorschlagsliste mit den Merkmalen der Befunde der aktuellen Befundliste vergleicht und die aktuelle Vorschlagsliste unter Berücksichtigung der Gefährlichkeit der Befunde der aktuellen Befundliste sortiert. So können Merkmale weiter vorn platziert werden, die für Befunde mit hohem Gefährdungspotential bzw. großer Schwere bedeutsam sind.

In allen Ausgestaltungen ist es bevorzugt, dass die Intensität (Ausprägung) eines Merkmals die Größe eines eingezeichneten Symbols bestimmt. Möglich ist es aber auch, dass die Intensität eines Merkmals die Farbe eines eingezeichneten Symbols oder die Helligkeit eines eingezeichneten Symbols bestimmt. Möglich ist es auch, dass in Abhängigkeit von den Eigenschaften eines Merkmals das entsprechende Symbol blinkend dargestellt oder mit einem Muster versehen wird.

In allen Ausgestaltungen ist es besonders bevorzugt, dass auch so genannte Negativmerkmale vorgebbar sind. Unter einem Negativmerkmal wird ein Merkmal verstanden, welches das Nicht - Vorhandensein des entsprechenden Merkmals definiert. Das Symptom "Fieber" ist z. B. ein wichtiges Symptom bei verschiedenen Krankheiten. Durch Ausschluss dieses Symptoms wird eine hohe Differenzierung erreicht. Wird nun das Merkmal "Fieber" als Negativmerkmal eingegeben, so bedeutet dies, dass der Patient kein Fieber hat, was gegebenenfalls viele Krankheiten ausschließt.

Solche Negativmerkmale können als spezielle Symbole gekennzeichnet werden, oder z.B. durch eine charakteristische farbige Einfassung oder Durchstreichung oder Durchkreuzung des das Merkmale charakterisierenden Symbols. Insbesondere werden sie grundsätzlich so dargestellt wie Positivmerkmale, aber beispielsweise mit einem zusätzlichen Kreis darum herum.

In allen Ausgestaltungen ist es bevorzugt, dass bei der Ausgabe eines Störungsbildes jedes Merkmal der zugehörigen Merkmalskonstellation aufgetragen wird, unabhängig davon ob ein solches Merkmal in der aktuellen Merkmalskonstellation vorhanden ist oder nicht, um insbesondere eine visuelle Bewertung der aktuellen Merkmalskonstellation mit den Merkmalskonstellationen der dargestellten Befunde der aktuellen Befundliste zu erleichtern.

Das erfindungsgemäße System dient zur Unterstützung der Diagnose eines wenigstens eine Störung aufweisenden Objektes und zur Auswahl wenigstens eines den Objektzustand wenigstens teilweise charakterisierenden Befundes, wobei in wenigstens einer Datenbank eine Anzahl von Befunden gespeichert ist. Insbesondere dient das System zur Durchführung eines zuvor beschriebenen Verfahrens.

Es ist wenigstens eine Anzeigeeinrichtung, wenigstens eine Recheneinrichtung, wenigstens eine Vergleichseinrichtung, wenigstens eine Speichereinrichtung und wenigstens eine Eingabeeinrichtung vorgesehen.

Wenigstens eine vordefinierte Merkmalskonstellation, wenigstens eine vordefinierte Objektliste mit gespeicherten Befunden und wenigstens eine vordefinierte Merkmalsliste mit möglichen Merkmalen sind in der Speichereinrichtung abgelegt. Mit der Recheneinrichtung sind aus der vordefinierten Befundliste eine aktuelle Befundliste und aus der vordefinierten Vorschlagsliste für Merkmale eine aktuelle Vorschlagsliste und aus der vordefinierten Merkmalskonstellation eine aktuelle Merkmalskonstellation ableitbar.

Jeder in der Speichereinrichtung gespeicherte Befund wird durch eine Schwere des Befundes und durch eine Merkmalskonstellation mehrerer Merkmale und durch eine graphisch auf der Anzeigeeinrichtung ausgebbare Wiedergabe der Merkmalskonstellation als visuelles Störungsbild definiert. Jedes Merkmal wird durch wenigstens einen Merkmalstyp und wenigstens eine Ausprägung definiert. Die Ausprägung gibt dabei die subjektive Wichtigkeit an.

Die Recheneinrichtung ist dazu eingerichtet und ausgebildet, in jedem Störungsbild jedes Merkmal durch ein Symbol zu repräsentieren. Die Koordinaten jedes Symbols werden in einer ersten Dimension durch den Merkmalstyp des Merkmals und in einer zweiten Dimension durch die Ausprägung des Merkmals bestimmt. Die Ausprägung des Merkmals wird subjektiv jedes Mal neu vergeben und gibt eine subjektiv empfundene Wichtigkeit in Hinblick auf die Ursache der Störung an.

Die Recheneinrichtung ist dazu eingerichtet und ausgebildet, eine dialogbasierte visuelle Modellierung des Objektzustandes durchzuführen. Der Objektzustand wird durch eine aktuelle Merkmalskonstellation beschrieben bzw. angenähert. Die aktuelle Merkmalskonstellation kann eines oder mehrere Merkmale umfassen und im Laufe der Durchführung mit mehr Merkmalen versehen werden. Jedes Merkmal weist wenigstens einen Merkmalstyp und wenigstens eine Ausprägung auf. Die aktuelle Merkmalskonstellation ist als Objektbild darstellbar, wobei die Recheneinrichtung dazu eingerichtet und ausgebildet ist, jedes Merkmal durch ein Symbol zu repräsentieren. Die Koordinaten jedes Symbols werden in einer ersten Dimension durch den Merkmalstyp des Merkmals und in einer zweiten Dimension durch die Ausprägung des Merkmals bestimmt.

Die Recheneinrichtung ist dazu ausgebildet und eingerichtet, die Modifizierung eines Merkmales der aktuellen Merkmalskonstellation zu erfassen und die aktuelle Merkmalskonstellation in der Speichereinrichtung abzulegen.

Die Recheneinrichtung ist dazu ausgebildet und eingerichtet, die aktuelle Merkmalskonstellation als aktuelles Objektbild automatisch und unmittelbar auf der Anzeigeeinrichtung graphisch auszugeben.

Die Vergleichseinrichtung ist dazu ausgebildet und eingerichtet, nach jeder Modifizierung eines Merkmals automatisch die aktuelle Merkmalskonstellation mit den Merkmalskonstellationen der Befunde der aktuellen Befundliste zu vergleichen und aus der aktuellen Befundliste Befunde zu entfernen, deren Übereinstimmung mit der aktuellen Merkmalskonstellation ein vorbestimmtes erstes Maß unterschreiten.

Die Vergleichseinrichtung ist dazu ausgebildet und eingerichtet, nach jeder Eingabe eines Merkmals automatisch die aktuelle Merkmalskonstellation mit den Merkmalskonstellationen von den in der Datenbank gespeicherten Befunden zu vergleichen und der aktuellen Objektliste gespeicherte Befunde hinzuzufügen, bei denen die Übereinstimmung der jeweiligen Merkmalskonstellation mit der aktuellen Merkmalskonstellation ein vorbestimmtes zweites Maß überschreitet.

Das erste und das zweite Maß können fest vorgegeben oder dynamisch gewählt werden. Das erste Maß kann gleich groß sein wie das zweite Maß oder insbesondere kleiner.

Die Recheneinrichtung ist dazu ausgebildet und eingerichtet, nach jeder Modifizierung eines Merkmals die aktuelle Befundliste automatisch zu sortieren, wobei die Ähnlichkeit der aktuellen Merkmalskonstellation mit der jeweiligen Merkmalskonstellation der gespeicherten Befunde und die Schwere des gespeicherten Befundes bei der Sortierung berücksichtigt werden.

Die Recheneinrichtung ist dazu ausgebildet und eingerichtet, von wenigstens einem Befund der aktuellen Befundliste die zugehörige Merkmalskonstellation als Störungsbild auf der Anzeigeeinrichtung aufzutragen, sofern die aktuelle Befundliste wenigstens einen Befund enthält.

Die Vergleichseinrichtung ist dazu ausgebildet und eingerichtet, nach jeder Modifizierung eines Merkmals die aktuelle Vorschlagsliste für Merkmale mit den Merkmalen der Befunde der aktuellen Befundliste automatisch zu vergleichen, und die aktuelle Vorschlagsliste neu zu sortieren.

Die Recheneinrichtung ist dazu ausgebildet und eingerichtet, die aktuelle Vorschlagsliste der Merkmale unmittelbar auszugeben.

Die Recheneinrichtung ist insbesondere dazu ausgebildet und eingerichtet, die aktuelle Merkmalskonstellation und insbesondere auch die aktuelle Vorschlagsliste und die aktuelle Befundliste in der Speichereinrichtung zu speichern.

Das erfindungsgemäße System hat viele Vorteile, da es eine einfache und übersichtliche Unterstützungshilfe bei der Auswahl von Merkmalskonstellationen bietet.

Insbesondere ist das erfindungsgemäße System als insbesondere mobiler und vorzugsweise gestengesteuerter Computer ausgeführt und weist eine berührungssensitive Oberfläche auf, mittels derer Merkmale ausgewählt und platziert werden können. Bevorzugt ist die Realisierung auch auf einem Smartphone.

Weitere Vorteile und Merkmale der vorliegenden Erfindung ergeben sich aus dem Ausführungsbeispiel, welches im Folgenden mit Bezug auf die beiliegenden Figuren erläutert wird.

In den Figuren zeigen:
- Fig. 1: die Oberfläche eines erfindungsgemäßen Systems;
- Fig. 2: den Darstellungsbereich des Systems nach Figur 1;
- Fig. 3: die Anzeigeeinrichtung des Systems nach Figur 1 nach der Eingabe von zwei Merkmalen;
- Fig. 4: die Anzeigeeinrichtung des Systems nach Figur 1 nach der Eingabe von drei Merkmalen; und
- Fig. 5: eine schematische Darstellung eines alternativen Darstellungsbereiches.

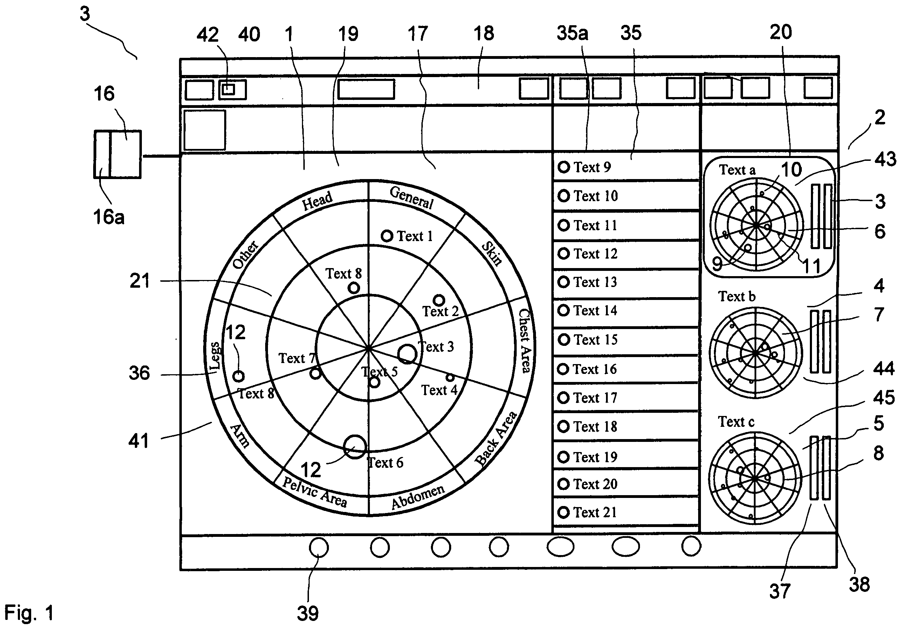

Mit Bezug auf die beiliegenden Figuren 1 bis 4 wird im Folgenden ein Ausführungsbeispiel eines erfindungsgemäßen Verfahrens mit einem erfindungsgemäßen System 30 erläutert. Das System 30 dient zur Unterstützung der Auswahl wenigstens eines Befundes bzw. einer Diagnose aus einer Gruppe von abgespeicherten Befunden und ist insbesondere für den Einsatz im medizinischen Bereich vorgesehen, kann aber auch bei der Analyse technischer Anlagen eingesetzt werden.

Das System 30 ermöglicht einem behandelnden Arzt und insbesondere einem Spezialisten auf einfache Art und Weise auch seltene Krankheiten über einen Mustervergleich von diagnostizierten Symptomen mit abgespeicherten Merkmalskonstellationen validierter Diagnosen im Blick zu behalten.

Dadurch wird es dem Benutzer möglich, die Auswahl zwischen zwei relativ gut zutreffenden Störungsbildern bzw. Merkmalskonstellationen zu treffen. Fehldiagnosen werden weitgehender vermieden, da durch die Übersicht über alle Symptome eines Krankheitsbildes und durch den Vergleich mit zuverlässig erfassten Diagnosen die Entscheidung sicherer wird, insbesondere auf medizinischen Gebieten, auf denen viele Faktoren zusammenwirken. Gegebenenfalls kann nach weiteren Untersuchungen die Entscheidung getroffen werden.

Weil beispielsweise das Symptom "Schwindel" für Erkrankungen des Gleichgewichtsorgans, Erkrankungen der Halswirbelsäule oder beispielsweise auch einen Schlaganfall stehen können, diese beiden Erkrankungen aber erkennbar unterschiedliche Merkmalskonstellationen aufweisen, bietet das erfindungsgemäße Verfahren mit einem graphischen Vergleich von Merkmalkonstellationen ein intuitive, schnelle und sehr effiziente Unterstützung bei der Deutung der Merkmalskonstellation.

Figur 1 zeigt ein System 30, welches hier beispielsweise als handgehaltener PC ausgeführt ist und welcher mit einer berührungssensitiven Oberfläche ausgestattet ist. Die Oberfläche des Systems 30 wird praktisch vollständig von einer Anzeigeeinrichtung 18 ausgefüllt, die hier im Ausführungsbeispiel auch gleich die Funktion der Eingabeeinrichtung 17 übernimmt. Gegebenenfalls ist es aber auch möglich, dass eine separate Eingabeeinrichtung oder dass mehrere separate Eingabeeinrichtungen 17 angeschlossen werden. Symbolisch ist eine Recheneinrichtung 16 eingezeichnet, die eine Vergleichseinrichtung 16a umfassen kann. Die Vergleichseinrichtung 16a kann auch separat vorgesehen sein. Die Recheneinrichtung 16 und die Vergleichseinrichtung 16a können auch über eine Netzwerkverbindung eingebunden werden.

Die Anzeige ist hier grundsätzlich in drei vertikale Abschnitte unterteilt, wobei der größte Abschnitt für den Darstellungsbereich 19 vorgesehen ist, der hier etwa die Hälfte der Breite einnimmt.

Im Anschluss daran findet sich rechts eine Merkmalsanzeige 35a mit einer Vorschlagsliste 35 für Merkmale, woran sich ein Auswahlbereich 20 anschließt. In der Merkmalsliste 35 werden Merkmale aufgelistet, die noch nicht auf dem Darstellungsbereich 19 angeordnet sind.

In dem System sind in einer Datenbank 42 gespeicherte Befunde 3 bis 5 vorgesehen, wobei grundsätzlich eine beliebige Vielzahl von Befunden gespeichert sein kann. Die Datenbank 42 kann lokal in einer Speichereinrichtung 40 vorgesehen oder über eine Netzwerkverbindung zugänglich sein. Jeder Befund 3 bis 5 repräsentiert im vorliegenden Ausführungsbeispiel eine Merkmalskonstellation einer Krankheit.

Jeder gespeicherte Befund 3 bis 5 wird durch genau eine Merkmalskonstellation 6 bis 8 definiert. Jede Merkmalkonstellation 6 bis 8 enthält eines oder mehrere Merkmale 9 bis 11. Grundsätzlich ist die Anzahl der Merkmale einer Merkmalkonstellation nicht begrenzt, sodass auch mehr als drei, vier, fünf oder mehr Merkmale zu einem gespeicherten Befund gehören können.

Jedes Merkmal wird durch einen Merkmalstyp 13 und durch eine Wichtigkeit bzw. Relevanz 14 sowie gegebenenfalls durch eine Intensität bzw. Ausprägung 15 gekennzeichnet. Einzelne oder alle Merkmale können auch noch weitere Eigenschaften haben.

In **Figur 1** ist in dem Darstellungsbereich 19 eine Merkmalkonstellation 21 graphisch repräsentiert wiedergegeben, wobei die Merkmalkonstellation 21 aus einer Vielzahl von Merkmalen 22 bis 24 besteht, die jeweils über Symbole 12 in den Darstellungsbereich 19 eingezeichnet sind. Die graphische Wiedergabe der aktuellen Merkmalkonstellation 21 zeigt das aktuelle Objektbild 41, welches während der Durchführung des Verfahrens bislang modelliert wurde. Wird ein neues Merkmal hinzugefügt oder aber ein schon hinzugefügtes Merkmal 22 bis 24 entfernt oder modifiziert, wird das Objektbild 41 automatisch und möglichst unmittelbar aktualisiert. Dadurch wird eine hohe Interaktivität erreicht, bei der der Benutzer sofort das Ergebnis seiner Modellierung sieht.

Effektiv wird der Darstellungsbereich 19 hier als kreisförmige Scheibe ausgeführt, wobei der Ort jedes Symbols 12 durch einen Winkel und einen Abstand vom Zentrum definiert wird. Der Winkel wird dabei durch den Merkmalstyp 13 vorgegeben und der Abstand vom Zentrum ergibt sich aus der Relevanz 14 des jeweiligen Merkmals 22 bis 24 für den Objektzustand 1 bzw. den Befund. Zu jedem Symbol 12 kann in dem Darstellungsbereich 19 ein Erläuterungstext "Text 1" etc. angegeben sein, der das Symptom beschreibt oder näher erläutert.

Die einzelnen Merkmale 22 bis 24 etc. werden von dem behandelnden Arzt in den Darstellungsbereich 19 gezogen und entsprechend dem Merkmalstyp 13 und der Wichtigkeit 14 positioniert. Die Ausprägung 14 wird von dem behandelnden Arzt subjektiv vergeben.

Wenn eine starke Ausprägung vergeben wird, hält der das Verfahren durchführende Experte das Merkmal für wichtig für den aktuellen Objektzustand 1 des Objektes. Das gilt ebenso bei der Diagnose von Patienten als auch bei der Untersuchung komplexer technischer Maschinen oder Anlagen.

Positioniert die Bedienperson ein Symbol 12 zentrumsnäher, so bedeutet dies, dass es nach seiner Auffassung wichtiger für die aktuelle Merkmalskonstellation 21 und somit Erkrankung ist. Wird das Symbol 12 radial weiter außerhalb positioniert, so gewichtet es der Arzt eher als Begleiterscheinung also als nicht so wichtig.

Neben dem Merkmalstyp 13 und der Relevanz 14 kann auch noch eine Intensität 15 für jedes Merkmal vergeben werden. Damit wird hier beispielsweise die Intensität eines Schmerzes bezeichnet. Beispielsweise kann der Patient über Kopfschmerzen klagen. Die Art der Schmerzen bestimmt den Merkmalstyp 13. Die Relevanz 14 vergibt der Arzt nach seiner Beurteilung der Wichtigkeit dieses Merkmals für die aktuelle Krankheit. Schließlich kann der Arzt durch die Größe des Symbols 12 kennzeichnen, wie stark die Schmerzen auf einer Schmerzskala sind.

Die Recheneinrichtung 16 ermittelt automatisch eine Befundliste 35 mit gespeicherten Befunden 3 bis 5, deren Merkmalskonstellation 6 bis 8 eine relativ hohe Ähnlichkeit mit der aktuell eingegebenen Merkmalskonstellation 21 aufweisen. Die Merkmalskonstellationen 6 bis 8 der gespeicherten Objekte 3 bis 5 werden graphisch in den Anzeigefelder 33 und 34 etc. jeweils separat abgebildet. Die Anzeigefelder 33 und 34 sind ein Teil des Auswahlbereichs 20 an der rechten Seite der Anzeigeeinrichtung 18. Dabei wird in analoger Weise zu der Merkmalkonstellation 21 jedes Merkmal 9 bis 11 der Merkmalkonstellation 6 bis 8 der gespeicherten Objekte 3 bis 5 jeweils als Symbol 12 graphisch wiedergegeben. Dadurch, dass die Anzeigeart der gespeicherten Objekte 3 bis 5 der Art der Anzeige der Merkmalkonstellation 21 entspricht, liegt eine hohe Vergleichbarkeit des Objektbildes 41 der aktuellen Merkmalkonstellation 21 und der Störungsbilder 43-45 der gespeicherten Merkmalkonstellationen 6 bis 8 vor. Aus Gründen der Übersichtlichkeit wird in der Regel darauf verzichtet, in dem Auswahlbereich 20 die Erläuterungstexte zu den einzelnen Merkmalen anzuzeigen.

So kann der Arzt durch einen visuellen Vergleich direkt feststellen, in welchem Bereich des Diagramms die Symbole 12 in welcher Art und Form angeordnet sind. Schon durch eine Häufigkeitsverteilung der Symbole 12 in unterschiedlichen Bereichen 36 kann der Arzt hilfreiche Rückschlüsse ziehen. Da vorzugsweise der Darstellungsbereich 19 in hier als Winkelsegmente ausgeführte Bereich 36 aufgeteilt ist, die beispielsweise unterschiedlichen Körperregionen zugeordnet sind, kann der Arzt schnell erkennen, wie sich die Symptome über den Körper des Patienten verteilen.

Die Aufteilung der Bereiche 36 kann insbesondere von dem medizinischen oder technischen Spezialgebiet abhängen, in den das System 30 verwendet wird. Besonderen Erfolg hat das System 30 bei der Unterstützung der Auswahl von Diagnosen, die auf Spezialgebieten durchgeführt werden und bei denen konventionelle Diagnosen aufgrund ihrer diffusen Symptome nur unzureichende Ergebnisse bringen.

Aufgrund der visuellen Vergleichbarkeit des Objektbildes 41 einer eingegebenen Merkmalkonstellation 21 mit den Störungsbildern 43 - 45 der gespeicherten Merkmalkonstellationen 6 - 8 wird hier eine einfache und dennoch zutreffende Auswahlmöglichkeit zur Verfügung gestellt. In der Darstellung nach Fig. 1 sind die im Darstellungsbereich 19 angeordneten Symptome mit dem erläuternden Text "Text 1" bis "Text 8" versehen. In der Vorschlagsliste 35 sind die dort aufgelisteten Merkmale mit dem erläuternden Text "Text 9" bis "Text 21" gekennzeichnet. Die Reihenfolge der Merkmale in der Merkmalsliste bzw. Vorschlagsliste 35 ergibt sich dynamisch. Das Merkmal mit dem höchsten Informationsgehalt wird vorzugsweise oben angeordnet.

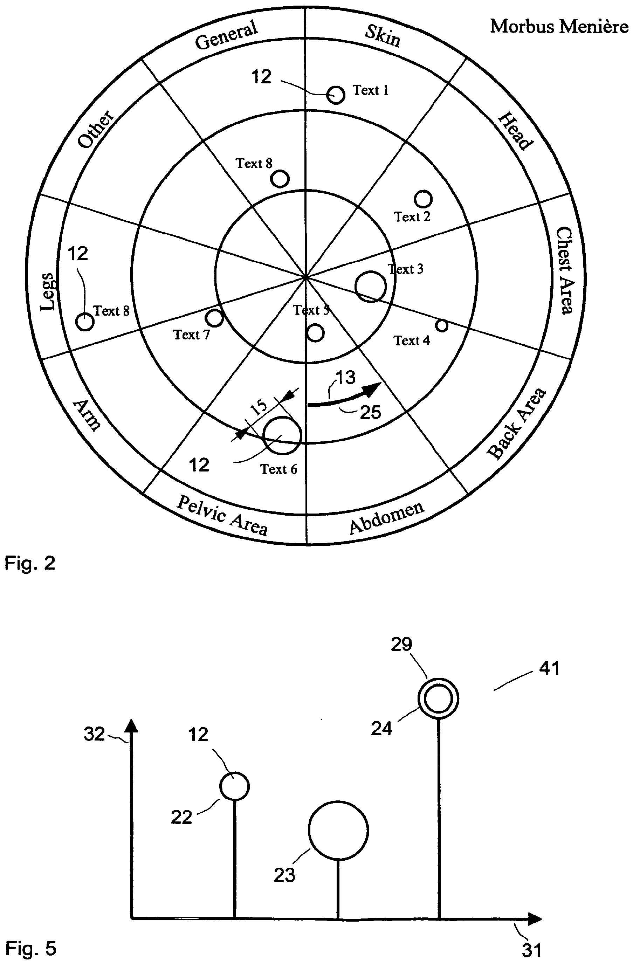

**Figur 2** zeigt in vergrößertem Maßstab den Darstellungsbereich 19 der Anzeigeeinrichtung 18, wobei eine Vielzahl von Symbolen 12 eingezeichnet ist, die entsprechende Symptome repräsentieren. Jedes Symbol 12 steht für einen Merkmalstyp 13, eine Relevanz 14 und eine entsprechende Intensität 15. Der Merkmalstyp 13 wird als Winkelkoordinate 31 in einer ersten Dimension 25 aufgetragen. Die Wichtigkeit 14 wird in einer radialen Koordinate 32 in einer zweiten Dimension 26 aufgetragen. Die Intensität 15 wird durch die Größe bzw. den Durchmesser eines Symbols 12 gekennzeichnet.

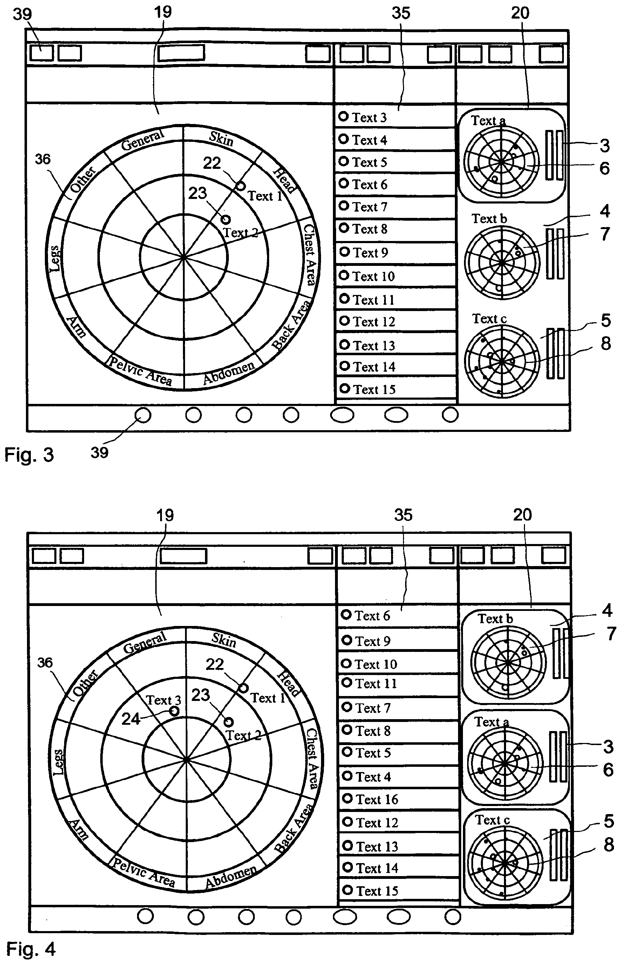

Mit Bezug auf die **Figuren 3 und 4** wird eine Vorgehensweise bei der Verwendung des Systems 30 erläutert. Der Arzt zieht von der Vorschlagsliste 35 ein Merkmal 22 bis 24 als Symbol 12 in den Darstellungsbereich 19.

Die winkelmäßige Anordnung des Symbols 12 auf dem Darstellungsbereich 19 ergibt sich durch den Merkmalstyp 13 des Merkmals. Der Arzt positioniert das Symbol 12 an einer radialen Stelle, die nach seiner Auffassung der Relevanz und somit hier der Wichtigkeit 13 für die vorliegende Erkrankung entspricht. Dabei werden hier in dieser Darstellung wichtige Symptome radial innen und weniger wichtige Symptome radial außen angeordnet. Das bedeutet, dass für die vorliegende Erkrankung das Merkmal 22 von dem behandelnden Arzt als unwichtiger als das Merkmal 23 angesehen wird Als Folge der beiden eingegebenen Merkmale 22 und 23 erfolgt im rechten Teil in dem Auswahlbereich 20 automatisch eine Auflistung möglicher relevanter Befunde 3 bis 5, wobei sich deren Reihenfolge nach der Übereinstimmung der jeweiligen Merkmalskonstellationen 6 bis 8 mit der Merkmalskonstellation 21 im linken Teil der Anzeigeeinrichtung 18 bzw. im Darstellungsbereich 19 ergibt. Gegebenenfalls ist wahlweise auch eine Überblendung möglich, bei der das Objektbild 41 einer eingegebenen Merkmalskonstellation 21 und ein Störungsbild 43-45 eines gespeichertes Befund 3 - 5 in dem Darstellungsbereich übereinander abgebildet werden. Eine Unterscheidung kann z.B. anhand der Farbe erfolgen.

Zwischen den Darstellungsbereichen 19 und dem Auswahlbereich 20 ist hier noch eine Merkmalsliste bzw. aktuelle Vorschlagsliste 35 vorgesehen, in der jene Symptome aufgelistet sind, die in den Krankheitsbildern aus der Auswahlliste 20 vorhanden sind, nicht aber im dem Darstellungsbereich 19. Insofern können die dort aufgelisteten Symptome bzw. Merkmale auch als Differenz-Merkmale bezeichnet werden. Die Liste wird dynamisch erzeugt. Weiter oben werden Symptome angeordnet, die eine höhere Differenzierung erlauben, also einen höheren Informationsgewinn bringen, als andere. Das bedeutet, dass als erstes Symptom auf der Liste 35 ein Symptom bzw. ein Merkmal 22-24 angeordnet wird, welches eine möglichst gute Unterscheidung zwischen den hier als möglicherweise relevant erkannten Diagnosen bzw. gespeicherten Befunden 3 bis 5 erlaubt.

Nach der Anordnung des Merkmals 24 mit dem Erläuterungstext "Text 3" auf dem Darstellungsbereich 19 ändert sich die Reihenfolge der Befunde 3 und 4 in dem Auswahlbereich 20, wie ein Vergleich der Figuren 3 und 4 zeigt. Das bedeutet, dass die Merkmalskonstellation 7 von Befund 4 nun eine höhere Übereinstimmung mit der Merkmalskonstellation 21 hat als die Merkmalskonstellation 6 von Befund 3.

Weiterhin hat sich auch die Reihenfolge der Merkmale auf der Vorschlagsliste 35 geändert. Das Merkmal mit der Erläuterung "Text 6" ist anschließend das oberste und nicht das erwartete Merkmal mit dem Erläuterungstext "Text 4", da sich durch die Mitberücksichtigung von Merkmal 24 insgesamt eine andere Gewichtung ergibt.

Der Arzt kann nun eines der Merkmale beispielsweise aus der Vorschlagsliste 35 in den Darstellungsbereichen 19 hineinziehen, sodass sich die in Figur 4 dargestellte Merkmalkonstellation 21 ergibt. Durch die neue Merkmalkonstellation kann sich die Reihenfolge der als möglicherweise relevant erachteten gespeicherten Befunde 3 bis 5 entsprechend ändern.

Jeder gespeicherte Befund 3, 4 bzw. dessen Merkmalkonstellation 6, 7 wird dabei jeweils in einem separaten Anzeigefeld 33, 34 dargestellt, um eine jeweils eindeutige Anzeige einer Merkmalkonstellation zu erhalten.

**Figur 5** zeigt eine Variante eines Darstellungsbereiches 19, bei dem die Anzeige in einem normalen X-Y-Diagramm auf Basis kartesischer Koordinaten erfolgt. Dabei wird über einer Koordinate 31 der Merkmalstyp 13 aufgetragen und senkrecht dazu auf der Koordinate 32 die Wichtigkeit 14.

Bei dieser Art der Darstellung wird keine Scheibenform gewählt, sondern ein normales kartesisches Koordinatensystem, welches ebenfalls ein charakteristisches Bild für jede Merkmalkonstellation ergibt.

In allen Fällen ist es möglich, dass neben Symptomen auch Negativsymptome eingegeben werden können. Dazu sind Negativmerkmale 29 vorgesehen, die das Nichtvorhandensein eines Symptoms kennzeichnen. Dieses kann ein besonders wichtiges Merkmal sein, da z. B. bei bestimmten Krankheiten das Vorhandensein von Fieber charakteristisch ist. Stellt der Arzt nun fest, dass der Patient kein Fieber hat, kann dies die Auswahl möglicher Diagnosen erheblich einschränken. Das Merkmal 24 ist hier als Negativmerkmal 29 dargestellt und weist einen doppelten Kreis als Symbol auf.

Insgesamt stellt die Erfindung ein einfaches System und ein vorteilhaftes Verfahren zur Verfügung, um einen Befund an Hand einer charakteristischen Konstellation von Merkmalen insbesondere in einem definierten graphischen Rahmen zu visualisieren, um so schnell und intuitiv eine Auswahl zu treffen. Der Benutzer wird die Darstellung und Aufbereitung im Laufe der Zeit verinnerlichen und somit ohne Nachzudenken intuitiv das System benutzen können.

Dabei steht die Zentrumsnähe für die Wichtigkeit 14, die Symbolgröße 15 für die Intensität und das Kreissegment 36 für eine Symptomklasse, während der Winkelgrad innerhalb des Kreissegments für einen Symptomtyp 13 steht. Damit ergibt sich ein System, ähnlich wie beim Lesen einer Landkarte.

Durch die charakteristische Anordnung von graphischen Symbolen 12 als Merkmalen 9-11 in einem vergebenen Rahmen kann ein Krankheitsbild in einer geringen räumlichen Größe charakteristisch und wieder erkennbar angeordnet werden.

Der visuell-konstellative Ansatz ermöglicht es, auch semantisch komplexe Gebilde, wie sogenannte Gestalten auf kleinstem Raum dazustellen. Der Betrachter hat trotz der Kleinheit einen guten Überblick.

### Bezugszeichenliste:

- 1: Objektzustand/Krankheit
- 2: Befundliste
- 3-5: gespeicherter Befund, Diagnose
- 6-8: Merkmalskonstellation, Symptomkonstellation
- 9-11: Merkmal, Symptom
- 12: Symbol
- 13: Merkmalstyp
- 14: Ausprägung, Relevanz, Wichtigkeit
- 15: Intensität, Stärke
- 16: Recheneinrichtung
- 16a: Vergleichseinrichtung
- 17: Eingabeeinrichtung
- 18: Anzeigeeinrichtung
- 19: Darstellungsbereich, Diagnosefeld
- 20: Auswahlbereich, Vergleichsanzeige
- 21: aktuelle Merkmalskonstellation, Krankheitsbild
- 22-24: Merkmal, Symptom
- 25: erste Dimension, Art des Symptoms
- 26: zweite Dimension, Relevanz
- 27: Schwere des Befundes
- 29: Negativmerkmal
- 30: System
- 31, 32: Koordinate
- 33, 34: Anzeigefeld
- 35: Vorschlagsliste
- 35a: Merkmalsanzeige
- 36: Bereich
- 37: Anzeige
- 38: Anzeige
- 39: Steuerelement
- 40: Speichereinrichtung
- 41: Objektbild
- 42: Datenbank
- 43-45: Störungsbild

## Patentansprüche

1. Computer-implementiertes Verfahren zur Unterstützung der Diagnose von Krankheiten eines Patienten und zur Auswahl eines die Krankheit (1) wenigstens teilweise charakterisierenden Befundes (3-5), wobei in wenigstens einer Datenbank (41) eine Anzahl von Befunden (3-5) gespeichert ist,
wobei jeder gespeicherte Befund (3-5) durch eine Schwere (27) des Befundes und eine Symptomkonstellation (6-8) mehrerer Symptome (9-11) und durch eine graphische Wiedergabe der Symptomkonstellation (6-8) als visuelles Störungsbild (43) beschrieben wird, wobei jedes Symptom (9-11) wenigstens einen Merkmalstyp (13) und wenigstens eine Ausprägung (14) aufweist, und wobei in jedem Störungsbild (43-45) jedes Symptom (22-24) durch ein Symbol (12) repräsentiert wird, dessen Koordinaten (31, 32) in einer ersten Dimension (25) durch den Merkmalstyp (13) des Symptoms (22-24) und in einer zweiten Dimension (26) durch Ausprägung (14) des Symptoms (22-24) bestimmt werden, wobei die Ausprägung durch eine subjektiv empfundene Wichtigkeit in Hinblick auf die störungsgenerierende Ursache festgelegt wird,
und wobei eine dialogbasierte visuelle Modellierung der Krankheit (1) erfolgt, wozu die Krankheit (1) durch ein aktuelles Krankheitsbild (21) beschrieben wird, das mehrere Symptome umfassen kann, wobei jedes Symptom wenigstens einen Merkmalstyp (13) und wenigstens eine Ausprägung (14) aufweist, und wobei das aktuelle Krankheitsbild (21) als Objektbild (41) dargestellt wird, wobei jedes Symptom durch ein Symbol (12) repräsentiert wird, dessen Koordinaten (31, 32) in einer ersten Dimension (25) durch den Merkmalstyp (13) des Symptom und in einer zweiten Dimension (26) durch die Ausprägung (14) des Symptoms bestimmt werden,
und wobei zur Unterstützung der Diagnose wenigstens eine Recheneinrichtung (16), wenigstens eine Vergleichseinrichtung (16a), wenigstens eine Eingabeeinrichtung (17) und wenigstens eine Anzeigeeinrichtung (18) vorgesehen sind und wobei die Anzeigeeinrichtung (18) wenigstens einen Darstellungsbereich (19) und wenigstens einen Auswahlbereich (20) umfasst,
wobei die folgenden Verfahrensschritte durchgeführt werden:
- aus einer vordefinierten Symptomkonstellation wird ein aktuelles Krankheitsbild (21) abgeleitet,
- aus einer vordefinierten Befundliste mit gespeicherten Befunden (3-5) wird eine aktuelle Befundliste (2) abgeleitet,
- aus einer vordefinierten Vorschlagsliste für Symptome wird eine aktuelle Vorschlagsliste (35) für Symptome abgeleitet,
und wobei die folgenden Verfahrensschritte in dieser oder einer anderen geeigneten Reihenfolge in einer Schleife automatisch wiederholt werden, bis ein Befund manuell festgelegt wird oder bis ein Störungsbild und damit der zugehörige Befund (3-5) ausgewählt wird oder bis das Verfahren abgebrochen wird:
a) mit der Eingabeeinrichtung (17) wird wenigstens ein Symptom (22-24) des aktuellen Krankheitsbildes (21) modifiziert, um die Krankheit (1) zu modellieren, und die modifizierte Symptomkonstellation wird als aktuelles Krankheitsbild (21) gespeichert und das aktuelle Krankheitsbild (21) wird als aktuelles Objektbild (41) automatisch und unmittelbar auf dem Darstellungsbereich (19) der Anzeigeeinrichtung (18) graphisch ausgegeben,
b) die Vergleichseinrichtung (16a) vergleicht nach jeder Modifizierung eines Symptoms automatisch das aktuelle Krankheitsbild (21) mit den gespeicherten Symptomkonstellationen (6-8) der Befunde (3-5) der aktuellen Befundliste (2) und entfernt unmittelbar aus der aktuellen Befundliste (2) Befunde (3-5), deren Übereinstimmung mit dem aktuellen Krankheitsbild (21) ein vorbestimmtes erstes Maß unterschreiten,
c) die Vergleichseinrichtung (16a) vergleicht nach jeder Modifizierung eines Symptoms automatisch das aktuelle Krankheitsbild (21) mit den Symptomkonstellationen von den in der Datenbank gespeicherten Befunden (3-5) und fügt unmittelbar der aktuellen Befundliste gespeicherte Befunde (3-5) hinzu, bei denen die Übereinstimmung der jeweiligen Symptomkonstellationen mit dem aktuellen Krankheitsbild (21) ein vorbestimmtes zweites Maß überschreitet,
d) die aktuelle Befundliste (2) wird nach jeder Modifizierung eines Symptoms mit der Recheneinrichtung (16) automatisch und unmittelbar sortiert, wobei die Ähnlichkeit des aktuellen Krankheitsbildes (21) mit der jeweiligen Symptomkonstellation (6-8) der gespeicherten Befunde (3-5) und die Schwere (27) des gespeicherten Befundes (3-5) bei der Sortierung berücksichtigt wird,
e) von wenigstens einem Befund (3-5) der aktuellen Befundliste wird die zugehörige Symptomkonstellation (6-8) als Störungsbild (41) auf dem Auswahlbereich der Anzeigeeinrichtung (18) aufgetragen, sofern die aktuelle Befundliste wenigstens einen Befund enthält,
f) die Vergleichseinrichtung (16a) vergleicht nach jeder Modifizierung eines Symptoms die aktuelle Vorschlagsliste (35) für Symptome mit den Symptomen der Befunde (3-5) der aktuellen Befundliste (2), und sortiert die aktuelle Vorschlagsliste (35) neu, und gibt die aktuelle Vorschlagsliste unmittelbar aus.

2. Verfahren nach Anspruch 1, wobei mit der Eingabeeinrichtung (17) wenigstens ein Symptom (22-24) der Krankheit (1) eingegeben, entfernt oder verändert und das aktuelle Krankheitsbild (21) entsprechend modifiziert wird.

3. Verfahren nach einem der vorhergehenden Ansprüche, wobei wenigstens ein Symptom (9-11) als weitere Eigenschaft eine Intensität (15) aufweist.

4. Verfahren nach einem der vorhergehenden Ansprüche, in welchem die Vergleichseinrichtung (16a) das aktuelle Krankheitsbild (21) mit den Symptomkonstellationen von den in der Datenbank gespeicherten Befunden (3-5) vergleicht und der aktuellen Befundliste (2) gespeicherte Befunde (3-5) hinzufügt, wenn die Übereinstimmung der jeweiligen Symptomkonstellation mit dem aktuellen Krankheitsbild (21) das vorbestimmte erste Maß unterschreitet, aber ein vorbestimmtes drittes Maß überschreitet, wenn das Störpotential des gespeicherten Befundes eine vorgegebene Schwelle überschreitet.

5. Verfahren nach einem der vorhergehenden Ansprüche, wobei die Gewichtung unterschiedlicher Symptome (9-11) vorgebbar ist.

6. Verfahren nach einem der vorhergehenden Ansprüche, wobei die Ausgabe der aktuellen Vorschlagsliste auf einer Merkmalsanzeige (35) vorgesehen ist.

7. Verfahren nach einem der vorhergehenden Ansprüche, wobei die Vergleichseinrichtung (16a) die Symptome der aktuellen Vorschlagsliste (35) mit den Symptomen der Befunde (3-5) der aktuellen Befundliste (2) vergleicht, und die aktuelle Vorschlagsliste unter Berücksichtigung der Gefährlichkeit der Befunde (3-5) der aktuellen Befundliste sortiert.

8. Verfahren nach einem der vorhergehenden Ansprüche, wobei der Merkmalstyp (13) als Winkelkoordinate (31) und die Wichtigkeit (14,32) als radialer Abstand und/oder die Intensität (15) als Symbolgröße (15) eingezeichnet wird.

9. Verfahren nach einem der vorhergehenden Ansprüche, wobei eine Modifizierung wenigstens eines Symptoms des aktuellen Krankheitsbildes (21) durch eine graphische Eingabe erfolgt.

10. Verfahren nach einem der vorhergehenden Ansprüche, wobei bei jeder Eingabe oder Modifizierung eines Symptoms die Ausprägung (14) neu definiert wird und eine subjektive Wichtigkeit dieses Merkmalstyps zur Erklärung der Krankheit angibt.

11. Verfahren nach einem der vorhergehenden Ansprüche, wobei Negativmerkmale (29) vorgebbar sind.

12. Verfahren nach einem der vorhergehenden Ansprüche, wobei die Farbe und/oder die Größe eines Symbols (12) eine Eigenschaft eines Symptoms (9-11) definieren.

13. Verfahren nach einem der vorhergehenden Ansprüche, wobei bei Ausgabe eines Störungsbildes (43-45) jedes Symptom der zugehörigen Symptomkonstellation (6-8) aufgetragen wird, unabhängig davon ob ein solches Symptom in dem aktuellen Krankheitsbild (21) vorhanden ist oder nicht, um insbesondere eine visuelle Bewertung des aktuellen Krankheitsbildes (21) mit den Symptomkonstellationen der dargestellten Befunde (3-5) der aktuellen Befundliste zu erleichtern.

14. Verfahren nach einem der vorhergehenden Ansprüche, wobei die aktuelle Vorschlagsliste (35) nach der Unterscheidungskraft der darin vorhandenen Symptome sortiert wird.

15. System (30) zur Unterstützung der Diagnose einer Krankheit und zur Auswahl wenigstens eines die Krankheit (1) wenigstens teilweise charakterisierenden (1) Befundes (3-5), wobei in wenigstens einer Datenbank eine Anzahl von Befunden (3-5) gespeichert ist, insbesondere zur Durchführung eines Verfahrens nach einem der vorhergehenden Ansprüche, wobei wenigstens eine Anzeigeeinrichtung, wenigstens eine Recheneinrichtung, wenigstens eine Vergleichseinrichtung (16a), wenigstens eine Speichereinrichtung (40), und wenigstens eine Eingabeeinrichtung (17) vorgesehen sind, und wobei die Anzeigeeinrichtung (18) wenigstens einen Darstellungsbereich (19) und wenigstens einen Auswahlbereich (20) umfasst,
wobei wenigstens eine vordefinierte Symptomkonstellation, wenigstens eine vordefinierte Objektliste mit gespeicherten Befunden (3-5) und wenigstens eine vordefinierte Merkmalsliste mit möglichen Symptomen in der Speichereinrichtung (40) abgelegt sind,
und wobei mit der Recheneinrichtung aus der vordefinierten Befundliste eine aktuelle Befundliste und aus der vordefinierten Vorschlagsliste für Symptome eine aktuelle Vorschlagsliste und aus der vordefinierten Symptomkonstellation ein aktuelles Krankheitsbild ableitbar ist,
wobei jeder in der Speichereinrichtung (40) gespeicherte Befund (3-5) durch eine Schwere (27) des Befundes und durch eine Symptomkonstellation mehrerer Symptome (9-11) und durch eine graphisch auf der Anzeigeeinrichtung ausgebbare Wiedergabe der Symptomkonstellation (6-8) als visuelles Störungsbild (43) definiert ist, und wobei jedes Symptom durch wenigstens einen Merkmalstyp und wenigstens eine die subjektive Wichtigkeit angebende Ausprägung (14) definiert ist, und wobei die Recheneinrichtung dazu eingerichtet und ausgebildet ist, in jedem Störungsbild (43-45) jedes Symptom (22-24) durch ein Symbol (12) zu repräsentieren, dessen Koordinaten (31, 32) in einer ersten Dimension (25) durch den Merkmalstyp (13) des Symptoms (22-24) und in einer zweiten Dimension (26) Ausprägung (14) des Symptoms (22-24) bestimmt werden, wobei die Ausprägung (14) des Symptoms (22-24) eine subjektiv empfundene Wichtigkeit in Hinblick auf die störungsgenerierende Ursache angibt, wobei die Recheneinrichtung dazu eingerichtet und ausgebildet ist eine dialogbasierte visuelle Modellierung der Krankheit (1) durchzuführen, bei der die Krankheit (1) durch ein aktuelles Krankheitsbild (21) beschrieben wird, das mehrere Symptome umfassen kann, wobei jedes Symptom wenigstens einen Merkmalstyp (13) und wenigstens eine Ausprägung (14) aufweist, und wobei die aktuelle Symptomkonstellation (21) als Objektbild (41) darstellbar ist, wobei die Recheneinrichtung dazu eingerichtet und ausgebildet ist jedes Symptom durch ein Symbol (12) zu repräsentieren, dessen Koordinaten (31, 32) in einer ersten Dimension (25) durch den Merkmalstyp (13) des Symptoms und in einer zweiten Dimension (26) durch die Ausprägung (14) des Symptoms bestimmt werden,
wobei die Recheneinrichtung dazu ausgebildet und eingerichtet ist, die Modifizierung eines Symptom (22-24) des aktuellen Krankheitsbildes zu erfassen und das aktuelle Krankheitsbild (21) in der Speichereinrichtung (40) abzulegen,
wobei die Recheneinrichtung dazu ausgebildet und eingerichtet ist, das aktuelle Krankheitsbild (21) als aktuelles Objektbild (41) automatisch und unmittelbar auf dem Darstellungsbereich (19) der Anzeigeeinrichtung (18) graphisch auszugeben,
wobei die Vergleichseinrichtung (16a) dazu ausgebildet und eingerichtet ist, nach jeder Modifizierung eines Symptoms automatisch das aktuelle Krankheitsbild (21) mit den Symptomkonstellationen der Befunde (3-5) der aktuellen Befundliste zu vergleichen und aus der aktuellen Befundliste Befunde (3-5) zu entfernen, deren Übereinstimmung mit des aktuellen Krankheitsbildes (21) ein vorbestimmtes erstes Maß unterschreiten,
wobei die die Vergleichseinrichtung (16a) dazu ausgebildet und eingerichtet ist, nach jeder Eingabe eines Symptoms automatisch das aktuelle Krankheitsbild (21) mit den Symptomkonstellationen von den in der Datenbank gespeicherten Befunden (3-5) zu vergleichen und der aktuellen Objektliste gespeicherte Befunde (3-5) hinzuzufügen, bei denen die Übereinstimmung der jeweiligen Symptomkonstellation mit dem aktuellen Krankheitsbild (21) ein vorbestimmtes zweites Maß überschreitet,
wobei die Recheneinrichtung (16) dazu ausgebildet und eingerichtet ist, nach jeder Modifizierung eines Symptoms die aktuelle Befundliste automatisch zu sortieren, wobei die Ähnlichkeit des aktuellen Krankheitsbildes mit der jeweiligen Symptomkonstellation der gespeicherten Befunde und die Schwere (27) des gespeicherten Befundes bei der Sortierung berücksichtigt wird,
wobei die Recheneinrichtung (16) dazu ausgebildet und eingerichtet ist, von wenigstens einem Befund (3-5) der aktuellen Befundliste die zugehörige Symptomkonstellation (6-8) als Störungsbild (41) auf dem Auswahlbereich der Anzeigeeinrichtung (18) aufzutragen, sofern die aktuelle Befundliste wenigstens einen Befund enthält,
wobei die Vergleichseinrichtung (16a) dazu ausgebildet und eingerichtet ist, nach jeder Modifizierung eines Symptoms die aktuelle Vorschlagsliste für Symptome mit den Symptomen der Befunde der aktuellen Befundliste automatisch zu vergleichen, und die aktuelle Vorschlagsliste neu zu sortieren,
und wobei die Recheneinrichtung dazu ausgebildet und eingerichtet ist, die aktuelle Vorschlagsliste der Symptome unmittelbar auszugeben
wobei die Recheneinrichtung dazu ausgebildet und eingerichtet ist, das aktuelle Krankheitsbild und insbesondere auch die aktuelle Vorschlagsliste und die aktuelle Befundliste in der Speichereinrichtung zu speichern.

## Claims

1. Computer-implemented method for aiding diagnosis of diseases of a patient and for selecting a finding (3-5) that characterises at least part of the disease (1), wherein a number of findings (3-5) is stored in at least one data base (41),
wherein each of the stored findings (3-5) is described by a severity (27) of the finding and a symptom constellation (6-8) of a number of symptoms (9-11) and by a graphic representation of the symptom constellation (6-8), as a visual pathology (43), wherein each symptom (9-11) shows at least one symptom type (13) and at least one expression (14), and wherein each symptom (22-24) in each pathology (43-45) is represented by a symbol (12) the coordinates (31, 32) of which are determined in a first dimension (25) by the symptom type (13) of the symptom (22-24) and in a second dimension (26) by expression (14) of the symptom (22-24), wherein the expression is determined by a subjectively perceived significance in respect of the disorder-generating cause,
and wherein a dialog-based, visual modelling of the disease (1) is carried out, to which end the disease (1) is described by a current clinical picture (21) which may comprise multiple symptoms, wherein each symptom shows at least one symptom type (13) and at least one expression (14), and wherein the current clinical picture (21) is represented as an object image (41), wherein each symptom is represented by a symbol (12) whose coordinates (31, 32) are determined in a first dimension (25) by the symptom type (13) of the symptom and in a second dimension (26) by the expression (14) of the symptom,
and wherein to aid diagnosis, at least one computer (16), at least one comparator (16a), at least one input device (17), and at least one display device (18) are provided, and wherein the display device (18) comprises at least one view area (19) and at least one selection area (20),
wherein the following process steps are carried out:
- a current clinical picture (21) is derived from a pre-defined symptom constellation,
- a current list of findings (2) is derived from a pre-defined list of findings containing stored findings (3-5),
- a current list of suggestions (35) for symptoms is derived from a pre-defined list of suggested symptoms,
and wherein the following process steps are automatically repeated in a loop in this or another suitable sequence until a finding is determined manually, or until a pathology and thus the pertaining finding (3-5) is selected or until the process is abandoned:
a) by way of the input device (17) at least one symptom (22-24) of the current clinical picture (21) is modified for modelling the disease (1), and the modified symptom constellation is stored as the current clinical picture (21), and the current clinical picture (21) is automatically and immediately graphically output as the current object image (41) on the view area (19) of the display device (18),
b) the comparator (16a) automatically compares, following each case of symptom modification, the current clinical picture (21) against the stored symptom constellations (6-8) of the findings (3-5) of the current list of findings (2) and immediately deletes from the current list of findings (2) those findings (3-5) showing a congruence with the current clinical picture (21) that remains beneath a predetermined, first value,
c) the comparator (16a) automatically compares, following each case of symptom modification, the current clinical picture (21) against the symptom constellations of the findings (3-5) stored in the data base and immediately adds to the current list of findings those stored findings (3-5) showing a congruence of the respective symptom constellation with the current clinical picture (21) that lies above a predetermined, second value,
d) the current list of findings (2) is automatically and immediately sorted by the computer (16) following each case of symptom modification, which sorting takes into account the similarity of the current clinical picture (21) with the respective symptom constellation (6-8) of the stored findings (3-5) and the severity (27) of the stored finding (3-5),
e) the symptom constellation (6-8) pertaining to at least one finding (3-5) of the current list of findings is plotted on the selection area of the display device (18) as a pathology (41), if the current list of findings includes at least one finding,
f) the comparator (16a) compares, following each case of symptom modification, the current list of suggestions (35) for symptoms against the symptoms of the findings (3-5) of the current list of findings (2), and re-sorts the current list of suggestions (35), and immediately outputs the current list of suggestions.

2. The method according to claim 1, wherein by means of the input device (17) at least one symptom (22-24) of the disease (1) is input, deleted or changed, and the current clinical picture (21) is modified correspondingly.

3. The method according to any of the preceding claims, wherein at least one symptom (9-11) comprises an intensity (15) as a further property.

4. The method according to any of the preceding claims, wherein the comparator (16a) compares the current clinical picture (21) against the symptom constellations of the findings (3-5) stored in the data base and adds stored findings (3-5) to the current list of findings (2), if the congruence of the respective symptom constellation with the current clinical picture (21) remains below the predetermined, first value but lies above a predetermined, third value if the disorder potential of the stored finding exceeds a predetermined threshold.

5. The method according to any of the preceding claims, wherein the weighting of different symptoms (9-11) can be predetermined.

6. The method according to any of the preceding claims, wherein the output of the current list of suggestions is provided on a symptom display (35).

7. The method according to any of the preceding claims, wherein the comparator (16a) compares the symptoms of the current list of suggestions (35) against the symptoms of the findings (3-5) of the current list of findings (2) and sorts the current list of suggestions, taking into account the seriousness of the findings (3-5) of the current list of findings.

8. The method according to any of the preceding claims, wherein the symptom type (13) is drawn in as an angular coordinate (31), and the significance (14, 32), as a radial distance and/or the intensity (15), as an icon (15).

9. The method according to any of the preceding claims, wherein a modification of at least one symptom of the current clinical picture (21) is carried out by graphic input.

10. The method according to any of the preceding claims, wherein with each input or modification of a symptom the expression (14) is re-defined, indicating a subjective significance of this symptom type for explaining the disease.

11. The method according to any of the preceding claims, wherein negative symptoms (29) can be specified.

12. The method according to any of the preceding claims, wherein the colour and/or the size of a symbol (12) define one property of a symptom (9-11).

13. The method according to any of the preceding claims, wherein upon outputting a pathology (43-45), each symptom of the pertaining symptom constellation (6-8) is plotted, independently of whether or not this symptom is present in the current clinical picture (21), so as to facilitate in particular a visual assessment of the current clinical picture (21) against the symptom constellations of the represented findings (3-5) of the current list of findings.

14. The method according to any of the preceding claims, wherein the current list of suggestions (35) is sorted by the distinctiveness of the symptoms contained therein.

15. System (30) for aiding diagnosis of a disease and for selecting at least one finding (3-5) that characterises at least part of the disease (1), wherein a number of findings (3-5) is stored in at least one data base, in particular for carrying out a method according to any of the preceding claims, wherein at least one display device, at least one computer, at least one comparator (16a), at least one memory device (40), and at least one input device (17) are provided, and wherein the display device (18) comprises at least one view area (19) and at least one selection area (20),
wherein at least one pre-defined symptom constellation, at least one pre-defined list of objects with stored findings (3-5) and at least one pre-defined list of symptoms including conceivable symptoms are stored in the memory device (40),
and wherein the computer allows to derive from the pre-defined list of findings, a current list of findings, and from the pre-defined list of suggested symptoms, a current list of suggestions, and from the pre-defined symptom constellation, a current clinical picture,
wherein each of the findings (3-5) stored in the memory device (40) is defined by a severity (27) of the findings and by a symptom constellation of a number of symptoms (9-11) and by a representation of the symptom constellation (6-8) that may be output graphically on the display device as a visual pathology (43), and wherein each symptom is defined by at least one symptom type and at least one expression (14) indicating the subjective significance, and wherein the computer is set up and configured to represent each symptom (22-24) in each pathology (43-45) by a symbol (12) whose coordinates (31, 32) are determined in a first dimension (25) by the symptom type (13) of the symptom (22-24) and in a second dimension (26), expression (14) of the symptom (22-24), wherein the expression (14) of the symptom (22-24) indicates a subjectively perceived significance in respect of the disorder-generating cause,
wherein the computer is set up and configured to perform a dialog-based, visual modelling of the disease (1), wherein the disease (1) is described by a current clinical picture (21) which may comprise multiple symptoms, wherein each symptom shows at least one symptom type (13) and at least one expression (14), and wherein the current symptom constellation (21) can be represented as an object image (41), wherein the computer is set up and configured to represent each symptom by a symbol (12) whose coordinates (31, 32) are determined in a first dimension (25) by the symptom type (13) of the symptom and in a second dimension (26), by the expression (14) of the symptom,
wherein the computer is configured and set up to capture the modification of a symptom (22-24) of the current clinical picture and to store the current clinical picture (21) in the memory device (40),
wherein the computer is configured and set up to automatically and immediately graphically output on the view area (19) of the display device (18), the current clinical picture (21) as the current object image (41),
wherein the comparator (16a) is configured and set up to automatically compare, following each case of symptom modification, the current clinical picture (21) against the symptom constellations of the findings (3-5) of the current list of findings and to delete from the current list of findings those findings (3-5) showing a congruence with the current clinical picture (21) that remains beneath a predetermined, first value,
wherein the comparator (16a) is configured and set up to automatically compare, following each entry of a symptom, the current clinical picture (21) against the symptom constellations of the findings (3-5) stored in the data base, and to add to the current list of objects those stored findings (3-5) showing a congruence of the respective symptom constellation with the current clinical picture (21) that lies above a predetermined, second value, wherein the computer (16) is configured and set up, following each case of symptom modification, to automatically sort the current list of findings, wherein the sorting takes into account the similarity of the current clinical picture with the respective symptom constellation of the stored findings and the severity (27) of the stored finding, wherein the computer (16) is configured and set up to plot on the selection area of the display device (18), as a pathology (41), the symptom constellation (6-8) pertaining to at least one finding (3-5) of the current list of findings, if the current list of findings includes at least one finding,
wherein the comparator (16a) is configured and set up, following each case of symptom modification, to automatically compare the current list of suggested symptoms against the symptoms of the findings of the current list of findings, and to re-sort the current list of suggestions,
and wherein the computer is configured and set up to immediately output the current list of suggested symptoms,
wherein the computer is configured and set up to store in the memory device the current clinical picture and in particular also the current list of suggestions and the current list of findings.

## Revendications

1. Procédé, mis en œuvre par ordinateur, d'aide au diagnostic de maladies d'un patient et de sélection d'un résultat (3-5) caractérisant au moins partiellement la maladie (1), un nombre de résultats (3-5) étant enregistré dans au moins une banque de données (41),
chaque résultat enregistré (3-5) étant décrit par une gravité (27) du résultat et une constellation de symptômes (6-8) de plusieurs symptômes (9-11), et par une reproduction graphique de ladite constellation de symptômes (6-8) à titre d'image visuelle du trouble (43), chaque symptôme (9-11) présentant au moins un type de caractéristique (13) et au moins une expression (14), et chaque symptôme (22-24) étant, dans chaque image du trouble (43-45'), représenté par un symbole (12) dont les coordonnées (31, 32) sont déterminées dans une première dimension (25) par le type de caractéristique (13) du symptôme (22-24) et, dans une deuxième dimension (26), par expression (14) du symptôme (22-24), ladite expression étant fixée par une importance ressentie subjectivement par rapport à la cause à l'origine du trouble,
et une modélisation, visuelle et qui repose sur le dialogue, de la maladie (1) étant dressée, pour laquelle la maladie (1) est décrite par un tableau clinique actuel (21) qui peut comprendre plusieurs symptômes, chaque symptôme présentant au moins un type de caractéristique (13) et au moins une expression (14), et ledit tableau clinique actuel (21) étant représenté à titre d'image du sujet (41), chaque symptôme étant représenté par un symbole (12) dont les coordonnées (31, 32) sont déterminées dans une première dimension (25) par le type de caractéristique (13) du symptôme et, dans une deuxième dimension (26), par l'expression (14) dudit symptôme,
et au moins un dispositif de calcul (16), au moins un dispositif de comparaison (16a), au moins un dispositif de saisie (17) et au moins un dispositif d'affichage (18) étant prévus pour l'aide au diagnostic, et le dispositif d'affichage (18) comprenant au moins une zone de représentation (19) et au moins une zone de sélection (20),
les étapes de procédé suivantes étant exécutées :
- déduction d'un tableau clinique actuel (21) à partir d'une constellation de symptômes prédéfinie,
- déduction d'une liste actuelle de résultats (2) à partir d'une liste de résultats prédéfinie dotée de résultats enregistrés (3-5),
- déduction d'une liste actuelle de propositions (35) de symptômes à partir d'une liste de propositions prédéfinie de symptômes,
et les étapes de procédés suivantes se répétant en boucle de manière automatique dans cet ordre ou dans un autre ordre adéquat jusqu'à ce qu'un résultat soit fixé manuellement ou jusqu'à ce qu'une image du trouble et, ainsi, le résultat correspondant (3-5) soient sélectionnés ou jusqu'à ce que le procédé soit interrompu :
a) le dispositif de saisie (17) permet de modifier au moins un symptôme (22-24) du tableau clinique actuel (21) pour effectuer la modélisation de la maladie (1), et la constellation de symptômes modifiée est enregistrée à titre de tableau clinique actuel (21) et ledit tableau clinique actuel (21) est affiché graphiquement, automatiquement et directement sur la zone d'affichage (19) du dispositif d'affichage (18), à titre d'image actuelle du sujet (41),
b) après chaque modification d'un symptôme, le dispositif de comparaison (16a) compare automatiquement le tableau clinique actuel (21) aux constellations de symptômes enregistrées (6-8) des résultats (3-5) de la liste actuelle de résultats (2), et retire directement de la liste actuelle de résultats (2) les résultats (3-5) dont la conformité au tableau clinique actuel (21) n'atteint pas un premier degré prédéfini,
c) après chaque modification d'un symptôme, le dispositif de comparaison (16a) compare automatiquement le tableau clinique actuel (21) aux constellations de symptômes des résultats enregistrés dans la banque de données (3-5),
et ajoute directement à la liste actuelle de résultats les résultats enregistrés (3-5) pour lesquels la conformité de la constellation respective de symptômes au tableau clinique actuel (21) dépasse un deuxième degré prédéfini,
d) après chaque modification d'un symptôme, le dispositif de calcul (16) effectue automatiquement et directement un classement dans la liste actuelle de résultats (2), la similitude du tableau clinique actuel (21) à la constellation respective de symptômes (6-8) des résultats enregistrés (3-5), et la gravité (27) du résultat enregistré (3-5) étant prises en compte dans ledit classement,
e) la constellation correspondante de symptômes (6-8) d'au moins un résultat (3-5) de la liste actuelle de résultats est appliquée à titre d'image du trouble (41) sur la zone de sélection du dispositif d'affichage (18), dès lors que la liste actuelle de résultats contient au moins un résultat,
f) après chaque modification d'un symptôme, le dispositif de comparaison (16a) compare la liste actuelle de propositions (35) de symptômes aux symptômes des résultats (3-5) de la liste actuelle de résultats (2), reclasse la liste actuelle de propositions (35) et émet directement la liste actuelle de propositions.

2. Procédé selon la revendication 1, le dispositif de saisie (17) permettant de saisir, de retirer ou de modifier au moins un symptôme (22-24) de la maladie (1), et de modifier en conséquence le tableau clinique actuel (21).

3. Procédé selon l'une quelconque des revendications précédentes, au moins un symptôme (9-11) présentant une intensité (15) à titre d'autre propriété.

4. Procédé selon l'une quelconque des revendications précédentes, dans lequel le dispositif de comparaison (16a) compare le tableau clinique actuel (21) aux constellations de symptômes des résultats enregistrés dans la banque de données (3-5)
et ajoute à la liste actuelle de résultats (2) des résultats enregistrés (3-5) si la conformité de la constellation respective de symptômes au tableau clinique actuel (21) n'atteint pas le premier degré prédéfini, mais dépasse un troisième degré prédéfini lorsque le potentiel d'interférence du résultat enregistré dépasse un seuil prédéfini.

5. Procédé selon l'une quelconque des revendications précédentes, la pondération de symptômes différents (9-11) pouvant être prédéfinie.

6. Procédé selon l'une quelconque des revendications précédentes, l'émission de la liste actuelle de propositions étant prévue sur un affichage de caractéristiques (35).

7. Procédé selon l'une quelconque des revendications précédentes, le dispositif de comparaison (16a) comparant les symptômes de la liste actuelle de propositions (35) aux symptômes des résultats (3-5) de la liste actuelle de résultats (2) et effectuant le classement de la liste actuelle de propositions en tenant compte de la dangerosité des résultats (3-5) de la liste actuelle de résultats.

8. Procédé selon l'une quelconque des revendications précédentes, le type de caractéristique (13) étant indiqué à titre de coordonnée angulaire (31), l'importance (14, 32) à titre d'écart radial et/ou l'intensité (15) à titre de taille de symbole (15).

9. Procédé selon l'une quelconque des revendications précédentes, une modification d'au moins un symptôme du tableau clinique actuel (21) s'effectuant par une saisie graphique.

10. Procédé selon l'une quelconque des revendications précédentes, l'expression (14) étant redéfinie à chaque saisie ou modification d'un symptôme, et indiquant une importance subjective de ce type de caractéristique pour expliquer la maladie.

11. Procédé selon l'une quelconque des revendications précédentes, des caractéristiques négatives (29) pouvant être prédéfinies.

12. Procédé selon l'une quelconque des revendications précédentes, la couleur et/ou la taille d'un symbole (12) définissant une propriété d'un symptôme (9-11).

13. Procédé selon l'une quelconque des revendications précédentes, chaque symptôme de la constellation de symptômes correspondante (6-8) étant affiché lors de l'émission d'une image du trouble (43-45), indépendamment du fait qu'un tel symptôme soit présent ou non dans le tableau clinique actuel (21), et ce pour faciliter une évaluation visuelle du tableau clinique actuel (21) avec les constellations de symptômes des résultats représentés (3-5) de la liste actuelle de résultats.

14. Procédé selon l'une quelconque des revendications précédentes, le classement de la liste actuelle de propositions (35) s'effectuant en fonction du caractère distinctif des symptômes qui y figurent.

15. Système (30) d'aide au diagnostic d'une maladie et de sélection d'au moins un résultat (3-5) caractérisant au moins partiellement la maladie (1), un nombre de résultats (3-5) étant enregistré dans au moins une banque de données, notamment pour mettre en œuvre un procédé selon l'une quelconque des revendications précédentes, au moins un dispositif d'affichage, au moins un dispositif de calcul, au moins un dispositif de comparaison (16a), au moins un dispositif d'enregistrement (40) et au moins un dispositif de saisie (17) étant prévus, et ledit dispositif d'affichage (18) comprenant au moins une zone d'affichage (19) et au moins une zone de sélection (20),
au moins une constellation prédéfinie de symptômes, au moins une liste prédéfinie de sujets avec résultats enregistrés (3-5) et au moins une liste prédéfinie de caractéristiques avec symptômes possibles étant stockées dans le dispositif d'enregistrement (40),
et le dispositif de calcul permettant de déduire une liste actuelle de résultat à partir d'une liste prédéfinie de résultats, une liste actuelle de propositions à partir de la liste prédéfinie de propositions de symptômes, et un tableau clinique actuel à partir de la constellation prédéfinie de symptômes,
chaque résultat (3-5) enregistré dans le dispositif d'enregistrement (40) étant défini par une gravité (27) du résultat et par une constellation de symptômes de plusieurs symptômes (9-11) et par une reproduction de la constellation de symptômes (6-8) affichable graphiquement sur le dispositif d'affichage, à titre d'image visuelle du trouble (43), et chaque symptôme étant défini par au moins un type de caractéristique et au moins une expression (14) indiquant l'importance subjective, et le dispositif de calcul étant conçu et réalisé pour représenter chaque symptôme (22-24) dans chaque image du trouble (43-45) par un symbole (12) dont les coordonnées (31, 32) sont déterminées dans une première dimension (25) par le type de caractéristique (13) du symptôme (22-24) et, dans une deuxième dimension (26), par expression (14) du symptôme (22-24), ladite expression (14) du symptôme (22-24) indiquant une importance subjective ressentie par rapport à la cause à l'origine du trouble,
le dispositif de calcul étant conçu et réalisé pour exécuter une modélisation visuelle, reposant sur le dialogue, de la maladie (1), dans lequel la maladie (1) est décrite par un tableau clinique actuel (21) qui peut comprendre plusieurs symptômes, chaque symptôme présentant au moins un type de caractéristique (13) et au moins une expression (14), et la constellation actuelle de symptômes (21) pouvant être représentée à titre d'image du sujet (41), le dispositif de calcul étant conçu et réalisé pour représenter chaque symptôme par un symbole (12) dont les coordonnées (31, 32) sont déterminées dans une première dimension (25) par le type de caractéristique (13) du symptôme et, dans une deuxième dimension (26), par l'expression (14) du symptôme,
le dispositif de calcul étant conçu et réalisé pour détecter la modification d'un symptôme (22-24) du tableau clinique actuel et pour stocker ledit tableau clinique actuel (21) dans le dispositif d'enregistrement (40),
ledit dispositif d'enregistrement étant conçu et réalisé pour émettre graphiquement le tableau clinique actuel (21) à titre d'image actuelle du sujet (41), automatiquement et directement sur la zone d'affichage (19) du dispositif d'affichage (18),
le dispositif de comparaison (16a) étant conçu et réalisé pour, après chaque modification d'un symptôme, comparer le tableau clinique actuel (21) automatiquement aux constellations de symptômes des résultats (3-5) de la liste actuelle de résultats, et pour retirer de la liste actuelle de résultats les résultats (3-5) dont la conformité au tableau clinique actuel (21) n'atteint pas un premier degré prédéfini,
ledit dispositif de comparaison (16a) étant conçu et réalisé pour, après chaque saisie d'un symptôme, comparer automatiquement le tableau clinique actuel (21) aux constellations de symptômes des résultats enregistrés dans la banque de données (3-5)
et pour ajouter à la liste actuelle de sujets les résultats enregistrés (3-5) pour lesquels la conformité de la constellation de symptômes respective au tableau clinique actuel (21) dépasse un deuxième degré prédéfini,
le dispositif de calcul (16) étant conçu et réalisé pour, après chaque modification d'un symptôme, effectuer automatiquement un classement de la liste actuelle de résultats, la similitude du tableau clinique actuel à la constellation de symptômes respective des résultats enregistrés et la gravité (27) du résultat enregistré étant prises en compte dans ledit classement,
le dispositif de calcul (16) étant conçu et réalisé pour appliquer sur la zone de sélection du dispositif d'affichage (18) la constellation correspondante de symptômes (6-8) d'au moins un résultat (3-5) de la liste actuelle de résultats à titre d'image du trouble (41), dès lors que la liste de résultats contient au moins un résultat,
le dispositif de comparaison (16a) étant conçu et réalisé pour, après chaque modification d'un symptôme, comparer automatiquement la liste actuelle de propositions de symptômes aux symptômes des résultats de la liste actuelle de résultats, et pour reclasser la liste actuelle de propositions,
et le dispositif de calcul étant conçu et réalisé pour émettre immédiatement la liste actuelle de propositions des symptômes,
ledit dispositif de calcul étant conçu et réalisé pour enregistrer le tableau clinique actuel et notamment aussi la liste actuelle de propositions et la liste actuelle de résultats dans le dispositif d'enregistrement.
